(19) Europäisches Patentamt
European Patent Office
Office européen des brevets

(11) Publication number: **0 126 206 B1**

# (12) EUROPEAN PATENT SPECIFICATION

(45) Date of publication of patent specification: **02.05.91**

(51) Int. Cl.[5]: **C12N 15/56**, C12N 15/81, C12N 15/10, C12P 21/02, C07H 21/04, C12P 7/06, C12N 9/34, C12N 1/16, //C12R1/85

(21) Application number: **84100834.5**

(22) Date of filing: **26.01.84**

Divisional application 87116406 filed on 06.11.87.

## (54) Glucoamylase cDNA.

(30) Priority: **28.01.83 US 461920**
**20.12.83 US 564078**
**20.12.83 US 563941**

(43) Date of publication of application:
**28.11.84 Bulletin 84/48**

(45) Publication of the grant of the patent:
**02.05.91 Bulletin 91/18**

(84) Designated Contracting States:
**BE CH DE FR GB LI NL SE**

(73) Proprietor: **CETUS CORPORATION**
**1400 Fifty-Third Street**
**Emeryville California 94608(US)**

(72) Inventor: **Nunberg, Jack H.**
**5933 Chabot Road**
**Oakland California 94618(US)**
Inventor: **Flatgaard, Jeffrey E.**
**2354 Hilgard Avenue**
**Berkeley California 94709(US)**
Inventor: **Innis, Michael Alan**
**3133 Carlsen Street**
**Oakland California 94602(US)**
Inventor: **Gelfand, David Harrow**
**6208 Chelton Drive**
**Oakland California 94611(US)**
Inventor: **Meade, James Henry**
**2540 Francis Drive**
**Pinole California 94564(US)**

**EP 0 126 206 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(56) References cited:
EP-A- 0 034 470
GB-A- 2 091 268

Recombinant DNA and genetic experimentation (1979), Pergamon Press, Editors J. Morgan and W.J. Whelan, pp. 123-128

CHEMICAL ABSTRACTS, vol. 89, n0. 15, 9 October 1978, p. 451, abstract no. 127732h, Columbus, Ohio, US; C BALL et al.:"Improvement in amyloglucosidase production following genetic recombination of Aspergillus niger strains"

CHEMICAL ABSTRACTS, vol. 97, no. 1, p. 167, abstract no. 1562z, Columbus, Ohio, US; A. NASIM et al.:"Use of recombinant DNA techniques for the development of amylolytic strains of Saccharomyces cerevisiae for alcohol production"

AGRICULTURAL AND BIOLOGICAL CHEMISTRY, vol. 47, November 1983, I. YAMASHITA et al.:"Molecular cloning of a glucoamylase-producing gene in the yeast Saccharomyces", pp. 2689-2692

(74) Representative: Vossius & Partner
Siebertstrasse 4 P.O. Box 86 07 67
W-8000 München 86(DE)

## Description

The present invention relates to a DNA sequence which encodes a glucoamylase protein of a fungus belonging to the filamentous Ascomycetes which is free of introns and is capable, when correctly combined with a cleaved expression vector, of expressing a protein having glucoamylase enzyme activity upon transformation of a yeast host organism by the vector, said protein being not produced naturally or natively by the yeast host organism.

Furthermore, the present invention relates to corresponding DNA expression vectors, to yeast host organisms transformed by said expression vector for processes for the production of said glucoamylase, to processes for the saccharification of starch or starch hydrolysates containing oligosaccharides and to processes for producing ethanol.

The following publications are referred to by corresponding number in this application:

1. Lineback, et al., Cereal Chemistry, 49:283 (1972).
1a. Svensson, et al., Carlsberg Res. Commun., 47:55 (1982).
1b. Svensson, et al., Abstract IV-27, XIth International Carbohydrate Symposium, Vancouver, British Columbia, Aug., 1982.
1c. Botstein, et al., in The Molecular Biology of the Yeast Saccharomyces-Metabolism and Gene Expression, ed. by Strathern, et al. (New York: Cold Spring Harbor Laboratory, 1982), p.607ff.
1d. Struhl, Nature, 305:391 (1983).
1e. European Pat. Application 81303155.6 (Publication 45573 dated February 10, 1982) to Stanford University.
2. Chirgwin, et al., Biochem., 18:5294 (1979).
3. Sehgal, Methods in Enzymology, 79:111 (1981), at p. 117.
4. Pelham, et al., Eur. J. Biochem., 67:247 (1976).
5. Maniatis, et al., Molecular Cloning: A Laboratory Manual, publ., Cold Spring Harbor, N.Y. (1982), pp. 344-349.
6. Ivarie, et al., Anal. Biochem., 97:24 (1979).
7. Chang, et al., Nature, 275:617 (1978).
8. Doel, et al., Nucleic Acids Res., 4:3701 (1977).
9. Southern, J. Mol. Biol., 98:503 (1975).
10. Sanger, et al., Proc. Nat. Acad. Sci. USA, 74:5463 (1977).
11. Messing, et al., Nucleic Acid Res., 9:309 (1981).
12. Maxam, et al., Proc. Nat. Acad. Sci. USA, 74:560 (1977).
13. Mount, Nucl. Acids Res., 10:459 (1982).
14. Langford, et al., Proc. Natl. Acad. Sci. USA, 80:1496 (1983).
15. Langford, et al., Cell, 33:519 (1983).
16. Holland, et al., J. Biol. Chem., 256:1385 (1981).
16a. Sutcliffe , Cold Spring Harbor Symposium on Quantitative Biology, 43: 77 (1978).
16b. Broach, et al., Gene, 8: 121 (1979).
16c. Beach, et al., Nature, 290: 140 (1981).
17a. Erlich, et al., J. Biol. Chem., 254:12,240 (1979).
17b. Erlich, et al., Inf. and Imm., 41:683 (1983).
18. Dewald, et al., in Methods in Enzymology, Vol. XXXII, Biomembranes, Part B, ed. by Fleischer et al. (New York: Academic Press, 1974), p.87-88.

The techniques of genetic engineering have been successfully applied to the pharmaceutical industry, resulting in a number of novel products. Increasingly, it has become apparent that the same technologies can be applied on a larger scale to the production of enzymes of value to other industries. The benefits of achieving commercially useful processes through genetic engineering are expected to include: (1) cost savings in enzyme production, (2) production of enzymes in organisms generally recognized as safe which are more suitable for food products, and (3) specific genetic modifications at the DNA level to improve enzyme properties such as thermal stability and other performance characteristics.

One important industrial application of genetic engineering involves improving the ability of industrial yeast strains to degrade complex carbohydrate substrates such as starch. Yeasts such as Saccharomyces cerevisiae which are suitable for alcoholic fermentation do not produce an enzyme capable of hydrolyzing starch to utilizable substrates. Currently, starch used as a food source in alcoholic fermentation must be saccharified, either chemically or enzymatically, in a separate process to produce utilizable substrates for the fermenting yeast.

"Recombinant DNA and Genetic Experimentation", Pergamon Press, 1975, p. 123-128, gives a general outline of the production of "valuable proteins" in alternate biological hosts. However, it cannot be taken from this reference how to improve glucoamylase expression results in yeasts.

It would thus be desirable to construct, by genetic recombination methods, a fermentation yeast such as S. cerevisiae which itself has the capacity to synthesize one or more enzymes capable of breaking down starch to utilizable substrates. European Pat. Appln. 0,034,470 discloses preparing recombinant DNA containing an amylase encoding gene by cleaving a bacterial donor microorganism to obtain DNA and inserting those fragments in a vector. The amylase enzymes produced from the DNA which are used to hydrolyze starch are preferably alpha-amylase, beta-amylase or a pullulanase.

Accordingly, in one aspect, the present invention is concerned with constructing a yeast fermentation host organism which contains, in recombinant form, a gene coding for a glucoamylase which is active in hydrolyzing starch at both alpha 1-4 and alpha 1-6 linkages to generate glucose.

The present invention generally concerns the construction of a glucoamylase gene which can be introduced in recombinant form into a yeast host and which can be advantageously expressed in this host.

According to one aspect of the invention, there is provided a modified DNA sequence coding for fungal glucoamylase protein or its single or multiple base substitutions, deletions, insertions or inversions, wherein said DNA sequence is derived from natural, synthetic or semi-synthetic sources and is capable, when correctly combined with a cleaved expression vector, of expressing a non-native protein having glucoamylase enzyme activity upon transformation by the vector of a host organism. Most preferably the expression vector is the plasmid pAC1 described further hereinbelow which has been cleaved at its HindIII site so that the sequence can be inserted at that site.

According to another aspect of the invention, it has been discovered that Aspergillus awamori cells, when grown under conditions which induce glucoamylase, contain a relatively high concentration of approximately 2.2 kilobase poly A RNA which is not detected in cells grown under noninducing conditions. The induced poly A RNA (mRNA) is capable of directing the synthesis, in a cell-free protein synthesizing system, of an unglycosylated polypeptide which has a molecular weight of between about 70,000 and 74,000 daltons. The polypeptide produced is immunologically reactive with antibodies prepared against A. awamori glucoamylase.

A radioactively labeled cDNA copy of the induced poly A RNA is produced which is used in hybridization studies to identify A. awamori genomic DNA fragments containing portions of the glucoamylase gene. The hybridization studies suggest that A. awamori contains a single glucoamylase gene.

Similarly, the cDNA is used to identify phage or plasmid vectors containing such genomic DNA fragments in recombinant form. The identified cloning vectors may be used in determining gene polynucleotide sequences and sequence homology with the cDNA.

When a HindIII fragment containing the A. awamori glucoamylase gene is inserted into a yeast, neither transcription nor translation in these heterologous hosts is detected.

The invention also provides for recombinant DNA expression vectors containing the DNA sequence. The vector is preferably one which is compatible with a selected foreign host organism, and permits expression of the gene in the host. The exogenous gene which is expressed may be genomic DNA, synthetic DNA or a cDNA obtained from a mRNA by use of reverse transcriptase.

A novel method for producing a glucoamylase gene containing the appropriate DNA sequence generally includes producing genomic digest fragments, providing a glucoamylase probe, using the probe to identify genomic digest fragments containing glucoamylase gene regions, molecularly cloning the identified genomic digest fragments, molecularly cloning partial cDNA, sequencing the genomic and cDNA clones, comparing the sequenced glucoamylase gene regions with all or a portion of the amino acid sequence of the mature glucoamylase enzyme to determine the existence and location of all the introns and exons in the genomic clones, and constructing a gene whose codon sequence is substantially identical to that of the genomic glucoamylase gene when the sequences comprising the introns are deleted.

In a preferred embodiment of the method, the glucoamylase probe is provided by selecting a fungal source capable of producing a level of glucoamylase, when grown on starch, which is at least about ten times that produced by the fungal species when grown on xylose or glycerol in the absence of starch, culturing cells of the selected fungus under conditions which induce secretion of glucoamylase into the culture medium, obtaining mRNA from the cultured cells, fractionating the mRNA obtained according to size, selecting an mRNA which is detectable as having a relatively high concentration with respect to the equivalent-sized mRNA produced by cells of the selected fungal species cultured under conditions which do not induce secretion of glucoamylase into the culture medium, and copying the selected mRNA to produce the glucoamylase probe.

In yet another embodiment of the invention is provided a host organism transformed with a DNA expression vector comprising a promoter fragment that functions in that host and a DNA segment having a modified DNA sequence coding for fungal glucoamylase protein, the DNA segment being in an orientation with the promoter fragment such that in the host it is expressed to produce a non-native glucoamylase protein.

The gene herein, when expressed in a host organism transformed by an expression vector comprising the gene, produces an enzyme having glucoamylase activity. Preferably the glucoamylase enzyme is produced as a preprotein with a signal sequence at its $NH_2$-terminus which is processed by the host organism during secretion.

In another embodiment, the invention relates to a process for producing glucose by saccharification of starch using a recombinant glucoamylase gene.

In another embodiment, the invention relates to a process for producing ethanol by simultaneous saccharification and fermentation which comprises growing, on a nonfermentable carbon source which is a substrate for glucoamylase enzyme, a host organism transformed by the DNA expression vector described above. The carbon source is preferably starch, soluble starch, maltose or isomaltose.

In yet another embodiment, the invention relates to a process for secreting any proteinaceous material extracellularly which comprises growing a host organism in a culture medium, which host is transformed by a DNA expression vector comprising a promoter fragment which function in the host organism, a signal sequence having substantially the following amino acid sequence:

MET SER PHE ARG SER LEU LEU ALA LEU SER GLY LEU VAL CYS THR
GLY LEU ALA ASN VAL ILE SER LYS ARG

and a DNA segment which codes for the proteinaceous material.

Preferably, the proteinaceous material is a protein which is normally secreted and most preferably it is glucoamylase. The vector may or may not contain a DNA segment which functions as an origin of replication, a selectable marker or a transcription terminator segment.

In the invention herein, the glucoamylase enzyme obtained when the heterologous gene is expressed in a yeast is found to by gylcosylated. In addition, a significant portion (eg., greater than 90%) of the glucoamylase is secreted in the media. Also, when the N-terminus of the non-native glucoamylase protein secreted in the media (having a purity of greater than 85%) was sequenced, the first 29 amino acids were found to be identical to the mature glucoamylase protein secreted by Aspergillus. The apparent molecular weight as determined by SDS polyacrylamide gel electrophoresis of the glucoamylase protein obtained herein is similar to that observed for the mature processed and glycosylated form of the native glucoamylase secreted by Aspergillus. Further, the carboxy terminal amino acid is identical to that of the large molecular weight form of glucoamylase produced by Aspergillus.

FIGURE 1 represents gel electrophoretic patterns showing in vitro translation of A. awamori mRNA from cells grown in medium containing xylose or starch as carbon source. Translation products were immunoprecipitated using rabbit anti-glucoamylase antibody (lane 1, xylose-grown cells; lane 3, starch-grown cells) or normal rabbit antibody (lane 2, xylose-grown cells; lane 4, starch-grown cells).

FIGURES 2A and 2B represent gel electrophoretic patterns identifying glucoamylase mRNA. In FIG. 2A, poly A-containing mRNA from cells grown in medium containing starch (lane 1) or xylose (lane 2) was analyzed by MeHgOH-agarose gel electrophoresis. Human and E. coli ribosomal RNAs provide molecular weight markers. The A. awamori ribosomal RNAs are indicated as '285' and '185'. The major 'induced' mRNA (arrow) was isolated from the gel and used to direct in vitro translation. In FIG. 2B, total translation products of reactions containing no exogenous mRNA (lane 1) or the isolated major 'induced' mRNA (lane 2) are shown. Immunoprecipitation of protein products in lane 2, using rabbit anti-glucoamylase antibody, is shown in lane 3.

FIGURE 3 shows a restriction endonuclease map of A. awamori genome surrounding the glucoamylase gene. The entire structural gene is contained within the 3.4 kilobase EcoRI fragment isolated from the Charon 4A library. The protein-encoding regions of the glucoamylase gene are indicated as solid boxes and the arrow indicates the direction and extent of transcription.

FIGURE 4 shows gel electrophoretic patterns where pGAR1 is used to hybridize to, and select, glucoamylase mRNA. Total A. awamori mRNA (lane 1) and mRNA isolated by virtue of hybridization to pGAR1 DNA (lane 2) was translated in vitro and the protein products are displayed. Protein products of lane 2 are immunoprecipitated using rabbit anti-glucoamylase antibody (lane 3) or normal rabbit antibody (lane 4).

FIGURE 5 illustrates primer extension to determine 5' termini of glucoamylase mRNA and the sequence which was determined. The products of primer extension at 42° C (lane 1) and 50° C (lane 2) are displayed on a sequencing gel in parallel with m13/dideoxynucleotide sequencing reactions of this region, utilizing the identical 15-mer primer. The sequence presented represents the glucoamylase mRNA sequence and is complementary to that read from the sequencing reactions shown.

FIGURE 6 illustrates a restriction map of the EcoRI fragment containing the genomic glucoamylase gene, where the shaded boxes under the sequence represent the exons or coding regions of the glucoamylase gene and the arrow represents the direction of mRNA transcription.

FIGURE 7 illustrates a plasmid map for pGAC9.

FIGURE 8 illustrates a plasmid map for pGC21.

FIGURE 9 illustrates plate assays for degradation of Baker's starch by various transformed yeast strains. The strains given below were streaked on minimal media containing histidine at 40 mg/l and 2% w/v Baker's starch. After 12 days incubation at 30° C the plates were stained with iodine vapors. The starch was stained purple, and the clear zones represent regions in which the starch has been hydrolyzed.

| Plate | Area of Plate | Yeast | Plasmid |
|---|---|---|---|
| 1 | a | C468 | pAC1 |
| | b | C468 | pGAC9 |
| | c | C468 | pGC21 |
| | d | C468 | pGC21 |
| 2 | a | C468 | pAC1 |
| | b | C468 | pGAC9 |
| | c | C468 | pGAC9 |
| | d | C468 | pGAC9 |
| 3 | a | H18 | pAC1 |
| | b | H18 | pGAC9 |
| | c | C303* | |
| | d | H18 | pGAC9 |

*C303 strain is S. diastaticus.

FIGURE 10 shows DEAE-Sepharose chromatography of glucoamylase produced by the recombinant yeast in a 10-liter fermentor.

FIGURE 11 shows gel electrophoretic patterns of: BioRad High Molecular Weight Protein Standards (lane 1), 25 μg A. awamori glucoamylase-I (lane 2 and 5), 25 μg A. awamori glucoamylase-II (lane 3 and 6), and 25 μg recombinant glucoamylase (lane 4 and 7). Lanes 1-4 were stained with Coomassie Blue stain and lanes 5-7 with Periodic Acid Schiff's stain.

The following terms used in the description are defined below:

"DNA sequence" refers to a linear array of nucleotides connected one to the other by phosphodiester bonds between the 3' and 5' carbons of adjacent pentoses.

"Modified DNA sequence" refers to a DNA sequence which is altered from the native glucoamylase DNA sequence such as by removing the introns from or modifying the introns of the native sequence. The examples illustrate sequences which are free of introns. Sequences substantially free of introns means greater than about 80% free.

"Glucoamylase enzyme activity" refers to the amount by which the enzyme in contact with an aqueous slurry of starch or starch hydrolysate degrades starch to glucose molecules.

"Single or multiple base substitutions and deletions, insertions and inversions" of the basic modified DNA sequence refer to degeneracy in the DNA sequence where the codons may be mutated or the deoxyribonucleotides may be derivatized to contain different bases or other elements, but the DNA sequence thus altered is still capable, on transformation in a host, of expressing glucoamylase protein.

"Fungal glucoamylase protein" refers to protein which is not derived from a bacterial source, but rather from a fungal source such as a strain from the genus Aspergillus. Thus, a modified DNA sequence coding for fungal glucoamylase protein signifies that the DNA is not derived from a bacterial donor microorganism.

"Non-native glucoamylase protein" refers to glucoamylase protein not produced naturally or natively by the microorganism used as the host.

"Nonfermentable carbon source which is a substrate for glucoamylase" refers to substrates for the glucoamylase enzyme which the host cannot ferment, such as starch, maltose, isomaltose and other starch derived oligosaccharides. Cellulose is not a substrate for glucoamylase and thus is not contemplated in this definition.

The present invention relates to a modified DNA sequence and an expression vector into which the gene has been introduced by recombinant DNA techniques, which, when transformed in a host organism, expresses glucoamylase. The modified DNA sequence may be derived from a natural, synthetic or semi-synthetic source. Preferably it is derived from a selected native fungal source which produces an induced level of glucoamylase which is at least about ten times its uninduced level. The induced level is that which is produced by the fungal species when grown on starch as a sole or primary carbon source, and the uninduced level, that observed when the fungal species is grown on glycerol or xylose.

The selected fungus for producing glucoamylase is suitably cultured under glucoamylase-induction conditions and a poly A RNA fraction from the cultured cells is isolated and size fractionated to reveal a glucoamylase mRNA present in a detectably higher concentration than in mRNA from uninduced cells. A glucoamylase cDNA is produced by copying the mRNA, using a reverse transcriptase.

The DNA sequence contemplated in the present invention is the sequence coding for the fungal glucoamylase (amylo-glucosidase) from the filamentous Ascomycetes, preferably from an Aspergillus species, and more preferably Aspergillus awamori. The native enzyme obtained from these sources is active in breaking down high molecular weight starch, and is able to hydrolyze alpha 1-6 branch linkages as well as alpha 1-4 chain linkages. Relatively high levels of the enzyme are produced and secreted in A. awamori cultures grown on starch and a variety of 6-carbon sugars, such as glucose.

Although the invention will be described with particular reference to A. awamori as a source of the DNA sequence, it is recognized that the invention applies to other fungal species of the Aspergillus genus which have an inducible glucoamylase. In particular, A. awamori glucoamylase appears to be similar, if not identical, to Aspergillus niger glucoamylase, as will be seen below.

The fungal species A. awamori was selected for detailed study. This fungal species, when grown on starch as a sole or primary carbon source, produces an amount of glucoamylase in the culture medium, based on measurable enzyme activity per cell dry weight, which is about 200 times that of cells grown on xylose or glycerol.

A. awamori, when grown on starch, produces and secretes at least two physically distinguishable glucoamylase enzymes. One of these enzymes, referred to as glucoamylase-I, has a molecular weight of about 74,900 daltons, as reported in Reference 1, and is glycosylated at some or all of the peptide serine and threonine residues. A second enzyme, glucoamylase-II, has a molecular weight of about 54,300 daltons, as reported in Reference 1, and is also glycosylated. It is noted that the sizes of the glycosylated glucoamylase protein given herein are only approximate, because glycoproteins are difficult to characterize precisely.

Several lines of evidence suggest that the two A. awamori glucoamylase enzymes are derived from a common polypeptide. Antibodies prepared against each enzyme form react immunospecifically with the other form, as will be seen below. The two enzymes have identical amino acid sequences in N-terminal fragments containing about 30 amino acids each. Further, these N-terminal sequences are identical to those in glucoamylase I and II forms from Aspergillus niger, and the two A. niger glucoamylase forms appear to be derived from a common polypeptide, as reported in Reference 1a. Experiments performed in support of the present application, discussed below, indicate that a single A. awamori glucoamylase gene codes for a single glucoamylase polypeptide precursor, which is very similar, if not identical, to that produced by A. niger.

According to one aspect of the invention, it has been discovered that cells of a selected fungal species, when grown under conditions which induce the secretion of glucoamylase into the culture medium, contain poly A RNA which is essentially undetectable in cells grown under noninducing conditions. The poly A RNA is capable of directing the synthesis, in a cell-free protein synthesizing system, of a polypeptide which is immunologically reactive with antibodies prepared against the glucoamylase from that fungal species.

Because the gene is not expressed in yeast hosts with its intact regulatory elements, it is necessary to delete or modify the introns and to exchange promoters so that the yeast will transcribe the gene, translate the mRNA, and produce an active glucoamylase.

The introns may be removed from the glucoamylase gene either by methods known in the literature for removing introns or by the simpler method described in section B of Example 2 below using specific restriction enzymes in various steps to create fragments which are then ligated together and using site-directed mutagenesis. In the mutagenesis technique the 5'-most intron of the glucoamylase gene is removed using a primer which is homologous to sequences on both sides of the intron and annealing this primer to a single-stranded DNA template of the glucoamylase genomic clone. The primer is then used to prime DNA synthesis of the complementary strand by extension of the primer on an M13 single-stranded phage DNA template. The resulting molecules were double-stranded circular molecules with single-stranded loops containing the intron sequence. When the molecules are transformed into cells, these loops may be excised, thereby removing the intron, but even without excision DNA replication will generate the correct progeny. If the introns are present in the gene, little or no glucoamylase enzyme is produced in a yeast in which the gene is expressed.

After the introns have been removed therefrom, the glucoamylase gene may be inserted by genetic recombination into a DNA expression vector, preferably a plasmid, which may then be used to transform a host organism. Suitable crganisms for this purpose include bacteria such as E. coli, viruses and yeasts. The host organism useful in this present invention must contain the appropriate genetic background for transformation thereof, i.e., the expression vector is compatible with the genetic background of the host strain. For example, the host recipient yeast strains C468 and H18, which are haploid S. cerevisiae laboratory strains employed in the following examples illustrating yeast hosts, are deficient in $\beta$-isopropylmalate dehydrogenase activity and therefore are complemented to leucine prototrophy by inserting into the expression vector the selectable marker $\beta$-isopropylmalate dehydrogenase (LEU 2). While the expression vector may by itself be capable of phenotypic selection by containing a selectable marker, it need not be so capable because the host can be screened or selected for the glucoamylase gene.

The preferred bacterial host herein is E. coli. The preferred yeast host strain herein is from a species of the genus Saccharomyces, preferably S. cerevisiae, S. uvarum, S. carlsbergensis, or mixtures or mutants thereof, more preferably a S. cerevisiae strain, and most preferably yeast strain C468 described further hereinbelow.

DNA expression or DNA transfer vectors suitable for transfer and replication have been described, e.g., in References 1c and 1d. Many of the yeast vectors in present use are derived from E. coli vectors such as pBR322. These references, 1c and 1d in particular, describe integrative transformation where the microorganism host is transformed with vectors with no origin of replication that integrate into the host chromosome and are maintained and replicated as part of that chromosome. In another embodiment of this invention the host may be transformed by autonomous replication where the vectors contain DNA segments which serve as origins of DNA replication in the host cell. Vectors containing autonomously replicating segments are also described in Reference 1e. Preferably the DNA segment capable of functioning as an origin of replication is from yeast. Two types of such origins of replication from yeast are: one derived from a naturally occurring yeast plasmid, commonly referred to as the 2 micron circle, which confers the ability to replicate independently of yeast chromosomal DNA, and one derived from the yeast chromosomal replication origin containing a replication origin sequence termed ars (autonomous replication sequence), which also provides autonomous replication capability.

The expression vector of this invention necessarily contains a promoter fragment which functions in microorganisms, i.e., the host being employed, as well as the modified DNA sequence coding for the fungal glucoamylase protein. The protein-encoding segment must be so oriented with the promoter fragment that in a microorganism host it is expressed to produce non-native glucoamylase. For bacteria such as E. coli a trp promoter is preferred. For yeast, a yeast promoter fragment is preferred. Among possible yeast promoter fragments for purposes herein are included, e.g., alcohol dehydrogenase (ADH-I), 3-phosphoglycerokinase (PGK), pyruvate kinase (PYK), triose phosphate isomerase (TPI), beta-isopropylmalate dehydrogenase (LEU2), glyceraldehyde 3-phosphate dehydrogenase (TDH), enolase I (ENO1), and the like. A preferred promoter fragment for purposes herein is from the enolase I gene.

The expression vector herein also preferably contains a microorganism transcription terminator segment following the segment coding for the protein, in a direction of transcription of the coding segment. Examples of possible transcription segments include the 3' segments of the above-listed genes. A preferred transcription terminator segment is from the enolase I gene.

A preferred host system consists of the S. cerevisiae yeast host strain C468 transformed by the plasmid pGAC9. This preferred transformed yeast strain was deposited with the American Type Culture Collection (ATCC), 12301 Parklawn Drive, Rockville, MD 20852 on November 17, 1983 and assigned ATCC Deposit Number 20,690. Another preferred host system consists of the E. coli host strain MH70 transformed by the plasmid pGC24, which transformant was deposited with the ATCC on December 16, 1983, and assigned ATCC Deposit Number 39,537.

The A. awamori glucoamylase signal sequence described below is shown to function in yeast for the efficient processing and secretion of glucoamylase from yeast. This sequence could also be used for the secretion of other proteins from yeast and preferably for the secretion of proteins that are normally secreted by their native host. Examples of such proteins include amylases, cellulases, proteases, interferons, lymphokines, insulin, and hormones.

The following examples serve to exemplify the practice of the invention. They are presented for illustrative purposes only, and should not be construed as limiting the invention in any way. Percentages are by weight unless specified otherwise. All experiments were performed following the NIH (U.S.A.) guidelines for containment.

EXAMPLES

All of the strains employed in the examples which have been deposited in depositories were deposited either with the U.S. Department of Agriculture Agricultural Research Service, National Regional Research Laboratories (NRRL) of Peoria, IL 61604 or with the American Type Culture Collection (ATCC) of Rockville, MD 20852. Each strain deposited with ATCC has the individual ATCC designations indicated in the examples pursuant to a contract between the ATCC and the assignee of this patent application, Cetus Corporation. The contract with ATCC provides for permanent availability of the progeny of these strains to the public on the issuance of the U.S. patent describing and identifying the deposits or the publications or upon the laying open to the public of any U.S. or foreign patent application, whichever comes first, and for availability of the progeny of these strains to one determined by the U.S. Commissioner of Patents and Trademarks to be entitled thereto according to 35 U.S.C. 122 and the Commissioner's rules pursuant thereto (including 37 CFR 1.14 with particular reference to 886 OG 638). The assignee of the present application has agreed that if any of these strains on deposit should die or be lost or destroyed when cultivated under suitable conditions, it will be promptly replaced on notification with a viable culture of the same strain. The NRRL deposits mentioned in the examples and not designated patent deposits have been freely available to the public prior to the filing date of this application. In the examples all parts and percentages are given by weight and all temperatures in degrees Celsius unless otherwise noted.

EXAMPLE 1

Determination of Nucleotide Sequence of Glucoamylase Gene

Experimentally, A. awamori cells were grown on either starch or xylose, as a primary source of carbon. The A. awamori cells were obtained from NRRL, Deposit Number 3112, and have been recently redeposited and assigned NRRL Deposit Number 15271. Fungal growth was initiated from a suspension of spores in water. The fungal cells were grown in an agitated culture at 30 °C for 2-5 days in a standard growth medium (1% w/v yeast extract, 0.01 M ammonium sulfate, 0.025 M potassium phosphate buffer, pH 7.0) together with 5% w/v of either starch or xylose. As noted above, cells grown on starch produced an amount of glucoamylase in the culture medium, based on measurable enzyme activity per cell dry weight, that was about 200 times that of cells grown on xylose.

Total cellular RNA was isolated from the fungal cultures by a guanidium thiocyanate/CsCl procedure essentially as described in Reference 2. Briefly, mycelia were wrung dry in cheese-cloth, frozen in liquid nitrogen, and ground to a powder in a mortar and pestle in liquid nitrogen. The cell powder was homogenized in a guanidium thiocyanate solution containing 10 mM adenosine: $VOSO_4$ complex. Following centrifugation to pellet cellular debris, CsCl was added to the homogenate and the RNA was pelleted through a pad of CsCl by a high speed centrifugation.

Poly A containing RNA (poly A RNA) was isolated from total RNA by two passages over oligo-dT cellulose, conventionally, and the poly A RNA was size-fractionated by agarose gel electrophoresis, according to standard procedures.

The induced poly A RNA was extracted from the agarose gel essentially as described in Reference 3. Briefly, the gel was melted and then frozen to release the RNA into solution. The solidified agarose was removed by centrifugation. The extracted poly A RNA was extracted with phenol and precipitated with ethanol.

To examine the translation products of the induced poly A RNA in a cell-free protein synthesizing system, antibodies against A. awamori glucoamylase were prepared. Glucoamylase-I and II from A. awamori were obtained from the filtrate of a culture of A. awamori cells grown under glucoamylase induction conditions. The filtrate was fractionated by ion exchange chromatography using a diethylaminoethylcellulose column. Elution with a pH gradient ranging from pH 8.0 to pH 3.0 yielded two protein peaks that showed glucoamylase activity. The enzyme that eluted at the lower pH included the larger glucoamylase-I, and the other peak, glucoamylase-II. Gel electrophoresis indicated that glucoamylase-II was pure, but that glucoamylase-I was not. Glucoamylase-I was purified further by molecular sieve chromatography on a cross-linked dextran, Sepharcryl ® S-200 column. Two peaks were observed, one of them containing glucoamylase-I, which was shown to be pure. For both enzyme forms, enzyme purity was established by polyacrylamide gel electrophoresis under non-detergent conditions, and by sodium dodecyl sulfate polyacrylamide gel electrophoresis (SDS-PAGE).

The two purified glucoamylase forms were used-separately to raise anti-glucoamylase antibodies in rabbits. Each of the two immunoglobulin G(IgG) antibody fractions produced were able to neutralize the glucoamylase activity of both glucoamylase forms. Further, Ouchterlony analysis of the two antibody fractions with the two enzyme forms indicated that each antibody reacts immunospecifically with both enzyme forms.

Poly A RNA from induced and noninduced A. awamori was used to direct the synthesis of radioactive-methionine-labeled polypeptides in a rabbit reticulocyte lysate kit obtained from New England Nuclear Co., Boston, Mass., and sold as Reticulocyte Lysate/Methionine L-[$^{35}$S]-Translation System. References 4 and 5 describe typical reticulocyte lysate systems. After a defined reaction period, aliquots of the lysate were removed and analyzed, either before or after reaction with anti-glucoamylase antibody or normal rabbit immunoglobulin G (IgG), by SDS-PAGE. The immunoreactive products were precipitated essentially according to the method described in Reference 6.

To determine the molecular basis for the accumulation of glucoamylase protein in starch-grown, but not xylose-grown, cultures of A. awamori, glucoamylase mRNA levels were examined. Total cellular mRNA was isolated and used to direct the synthesis of A. awamori protein in a rabbit reticulocyte lysate system. The translation products were immunoprecipitated using rabbit anti-glucoamylase antibody (lane 1, xylose-grown cells; lane 3, starch-grown cells) or normal rabbit antibody (lane 2, xylose-grown cells; lane 4, starch-grown cells). The results are shown in FIG.1 and demonstrate the presence of translatable glucoamylase mRNA in RNA from starch-grown cells. In contrast, no glucoamylase mRNA was detected in xylose-grown cells. This correlates with the 200-fold difference in glucoamylase protein observed in culture supernatants of these cells. Thus, the accumulation of glucoamylase protein in starch-grown cultures appears to result from a comparable increase in translatable glucoamylase mRNA.

MeHgOH-agarose gel electrophoresis of mRNA from starch-grown cells revealed a major approximately 2.2 kilobase mRNA (indicated by an arrow), which was absent in mRNA from xylose-grown cells [FIG. 2A]. It appeared likely that this predominant 'induced' mRNA represented the mRNA of the highly expressed, 'induced' glucoamylase. To identify the 'induced' mRNA, the approximately 2.2-kilobase mRNA band was eluted from a gel and translated in the rabbit reticulocyte lysate system. Immunoprecipitation of the protein product with rabbit anti-glucoamylase antibody demonstrated the presence of mRNA encoding glucoamylase within the approximately 2.2-kilobase 'induced' mRNA band (FIG. 2B).

According to one aspect of the invention, isolated glucoamylase mRNA from the selected fungal species was used to produce a glucoamylase cDNA by reverse transcription of the mRNA. Experimentally, induced poly A RNA from A. awamori was pretreated with 10 mM MeHgOH to denature the RNA, and then introduced into a reaction containing oligo-dT as a primer and 2 mM adenosine: $VOSO_4$ as an RNAse inhibitor. The reader is referred to Reference 7 for a discussion of this general technique. Following cDNA synthesis, the poly A RNA was destroyed by treatment with NaOH. The synthesized cDNA was size fractionated by gel electrophoresis to separate the full-length cDNA from incompletely formed fragments. A typical gel electrophoretic pattern of the cDNA-fraction showed a single detectable band in the approximately 2.2 kilobase size region.

The induced glucoamylase mRNA and the cDNA produced therefrom were radiolabeled to provide probes for identifying genomic DNA fragments containing all or portions of the homologous glucoamylase gene. The cDNA may be labeled readily by performing its synthesis in the presence of radiolabeled nucleotides.

The basic method used for radiolabeling mRNA is discussed in Reference 8. In one example, induced poly A RNA from A. awamori was partially degraded, using sodium hydroxide to generate fragments containing 5'-OH groups. These fragments were subsequently phosphorylated with radioactive-phosphate ($^{32}$P)-ATP using a polynucleotide kinase. The $^{32}$P-labeled RNA fragments span the entire length of the isolated RNA, and are thus advantageous for use as probes for genomic DNA fragments containing end portions of the glucoamylase gene.

Total genomic DNA isolated from A. awamori was digested to completion with each of a number of restriction endonucleases. The fragments were size-fractionated by gel electrophoresis and hybridized to one of the above RNA or cDNA probes by the Southern blot method (Reference 9). Details of this method are found generally by Reference 5, at page 387. Briefly, a prehybridization step was performed at 42° C for 24 hours, using a five-times concentrate of standard saline citrate (0.15M sodium chloride, 0.015M trisodium citrate). This was followed by a hybridization step carried out at 420° C for 24 hours, using a two-times concentrate of the standard saline citrate. In the studies involving A. awamori genomic DNA, several of the endonucleases used--including HindIII, XhoI, Bc1I, and PvuI--generated only one fragment which hybridized to the above A. awamori labeled RNA or cDNA probes. Some of the single gene fragments are in the same size range as the RNA transcript, strongly indicating that A. awamori contains only one gene which codes for the glucoamylase polypeptide. EcoRI generated a 3.4 kilobase fragment which hybridized to the labeled cDNA.

The A. awamori genomic DNA fragments produced by digestion with EcoRI were spliced, by conventional techniques, into a lambda Charon 4A phage vector. The library of EcoRI fragments were screened for recombinants which hybridized to the A. awamori glucoamylase cDNA. Hybridizing plaques were purified, and all contained a common 3.4 kilobase EcoRI fragment which hybridized to the glucoamylase cDNA probe. This 3.4 kilobase EcoRI fragment was then subcloned into the EcoRI site of a pACYC184 plasmid (ATCC Deposit No. 37,033), producing a recombinant plasmid which is designated herein as pGAR1. A sample of E. coli K12 strain MM294 transformed with pGAR1 was deposited in the American Type Culture Collection, 12301 Parklawn Drive, Rockville, MD 20852, USA on December 2, 1983, and has been assigned ATCC Number 39,527. Subsequent libraries were screened using pGAR1 as probe. Approximately 20 kilobases of A. awamori genomic DNA surrounding the glucoamylase gene was isolated from EcoRI, HindIII and BglII libraries. A composite restriction map of this 20 kilobase region is shown in FIG. 3; the EcoRI fragment insert is expanded. The locations of the cleavage sites of the designated restriction endonucleases were determined by digesting the plasmids with selected combinations of the endonucleases, and size-fractionating the fragments obtained, according to known methods. The five solid rectangles represent sequenced protein-encoding regions of the glucoamylase gene. The direction of transcription of the mRNA is indicated by the 5' to 3' line.

The plasmid pGAR1 was confirmed to contain glucoamylase gene sequences by virtue of its ability to hybridize to and select A. awamori glucoamylase mRNA sequences. pGAR1 DNA was immobilized onto nitrocellulose and hybridized to total A. awamori mRNA. The selected mRNA was translated in vitro, and the products were identified by immunoprecipitation with rabbit anti-glucoamylase antibody. The results, shown in FIGURE 4, confirm the identification of pGAR1, and thus of the approximately 2.2 kilobase "induced" mRNA, as encoding glucoamylase. In FIGURE 4 total A. awamori mRNA (lane 1) and mRNA isolated by virtue of hybridization to pGAR1 DNA (lane 2) was translated in vitro and the protein products are displayed. Protein products of lane 2 were immunoprecipitated using rabbit antiglucoamylase antibody (lane 3) or normal rabbit antibody (lane 4).

Subclone pGAR1 containing the A. awamori glucoamylase gene was digested substantially to completion with various restriction enzymes whose sequences are included within the EcoRI fragment (i.e., those in FIGURE 6), and several of the fragments were subcloned into M13 vectors M13mp8 and M13mp9. These bacteriophage vectors are available from Bethesda Research Laboratories, P.O. Box 6009, Gaithersburg, MD 20877.

The fragments of the glucoamylase genomic region subcloned into the vectors M13mp8 and M13mp9 were sequenced by the dideoxynucleotide chain termination method described in References 10 and 11. Portions of the sequence were confirmed by the Maxam-Gilbert sequencing technique (Reference 12). The entire sequence of the 3.4 kilobase EcoRI fragment is shown in Table I below.

## TABLE I

| | |
|---|---|
| GAATTCAAGC TAGATGCTAA GCGATATTGC ATGGCAATAT GTGTTGATGC | 50 |
| ATGTGCTTCT TCCTTCAGCT TCCCCTCGTG CAGATGAAGG TTTGGCTATA | 100 |
| AATTGAAGTG GTTGGTCGGG GTTCCGTGAG GGGCTGAAGT GCTTCCTCCC | 150 |
| TTTTAGACGC AACTGAGAGC CTGAGCTTCA TCCCCAGCAT CATTACACCT | 200 |
| CAGCA ATG TCG TTC CGA TCT CTA CTC GCC CTG AGC GGC CTC GTC | 244 |

MET SER PHE ARG SER LEU LEU ALA LEU SER GLY LEU VAL -12

TGC ACA GGG TTG GCA AAT GTG ATT TCC AAG CGC GCG ACC TTG GAT 289
CYS THR GLY LEU ALA ASN VAL ILE SER LYS ARG Ala Thr Leu Asp 4

TCA TGG TTG AGC AAC GAA GCG ACC GTG GCT CGT ACT GCC ATC CTG 334
Ser Trp Leu Ser Asn Glu Ala Thr Val Ala Arg Thr Ala Ile Leu 19

AAT AAC ATC GGG GCG GAC GGT GCT TGG GTG TCG GGC GCG GAC TCT 379
Asn Asn Ile Gly Ala Asp Gly Ala Trp Val Ser Gly Ala Asp Ser 34

GGC ATT GTC GTT GCT AGT CCC AGC ACG GAT AAC CCG GAC T 419
Gly Ile Val Val Ala Ser Pro Ser Thr Asp Asn Pro Asp 47

gtatgtttcg agctcagatt tagtatgagt gtgtcattga ttgattgatg 469
--

ctgactggcg tgtcgtttgt tgtag AC TTC TAC ACC TGG ACT CGC GAC 517
Tyr Phe Tyr Thr Trp Thr Arg Asp 55

TCT GGT CTC GTC CTC AAG ACC CTC GTC GAT CTC TTC CGA AAT GGA 562
Ser Gly Leu Val Leu Lys Thr Leu Val Asp Leu Phe Arg Asn Gly 70

GAT ACC AGT CTC CTC TCC ACC ATT GAG AAC TAC ATC TCC GCC CAG 607
Asp Thr Ser Leu Leu Ser Thr Ile Glu Asn Tyr Ile Ser Ala Gln 85

GCA ATT GTC CAG GGT ATC AGT AAC CCC TCT GGT GAT CTG TCC AGC 652
Ala Ile Val Gln Gly Ile Ser Asn Pro Ser Gly Asp Leu Ser Ser 100

GCT
Ala
GGC GGT CTC GGT GAA CCC AAG TTC AAT GTC GAT GAG ACT GCC TAC 697
Gly Gly Leu Gly Glu Pro Lys Phe Asn Val Asp Glu Thr Ala Tyr 115

GCT CTG AGA
ACT GGT TCT TGG GGA CGG CCG CAG CGA GAT GGT CCG ~~GCC TCT GAG~~ 742
Thr Gly Ser Trp Gly Arg Pro Gln Arg Asp Gly Pro Ala ~~Ser Glu~~ 130
Leu Arg

GCA
~~AGA~~ ACT GCT ATG ATC GGC TTC GGG CAA TGG CTG CTT gtatgttctc 788
~~Arg~~ Thr Ala Met Ile Gly Phe Gly Gln Trp Leu Leu 142
Ala

cacccccttg cgtctgatct gtgacatatg tagctgactg gtcag GAC AAT 839
Asp Asn 144

GGC TAC ACC AGC ACC GCA ACG GAC ATT GTT TGG CCC CTC GTT AGG 884
Gly Tyr Thr Ser Thr Ala Thr Asp Ile Val Trp Pro Leu Val Arg 159

AAC GAC CTG TCG TAT GTG GCT CAA TAC TGG AAC CAG ACA GGA TAT 929
Asn Asp Leu Ser Tyr Val Ala Gln Tyr Trp Asn Gln Thr Gly Tyr 174

G gtgtgtttgt tttatttaa atttccaaag atgcgccagc agagctaacc 980
---

cgcgatcgca g AT CTC TGG GAA GAA GTC AAT GGC TCG TCT TTC 1023
Asp Leu Trp Glu Glu Val Asn Gly Ser Ser Phe 185

```
TTT ACG ATT GCT GTG CAA CAC CGC GCC CTT GTC GAA GGT AGT GCC   1068
Phe Thr Ile Ala Val Gln His Arg Ala Leu Val Glu Gly Ser Ala    200

TTC GCG ACG GCC GTC GGC TCG TCC TGC TCC TGG TGT GAT TCT CAG   1113
Phe Ala Thr Ala Val Gly Ser Ser Cys Ser Trp Cys Asp Ser Gln    215

GCA CCC GAA ATT CTC TGC TAC CTG CAG TCC TTC TGG ACC GGC AGC   1158
Ala Pro Glu Ile Leu Cys Tyr Leu Gln Ser Phe Trp Thr Gly Ser    230

TTC ATT CTG GCC AAC TTC GAT AGC AGC CGT TCC GGC AAG GAC GCA   1203
Phe Ile Leu Ala Asn Phe Asp Ser Ser Arg Ser Gly Lys Asp Ala    245

AAC ACC CTC CTG GGA AGC ATC CAC ACC TTT GAT CCT GAG GCC GCA   1248
Asn Thr Leu Leu Gly Ser Ile His Thr Phe Asp Pro Glu Ala Ala    260

TGC GAC GAC TCC ACC TTC CAG CCC TGC TCC CCG CGC GCG CTC GCC   1293
Cys Asp Asp Ser Thr Phe Gln Pro Cys Ser Pro Arg Ala Leu Ala    275

AAC CAC AAG GAG GTT GTA GAC TCT TTC CGC TCA ATC TAT ACC CTC   1338
Asn His Lys Glu Val Val Asp Ser Phe Arg Ser Ile Tyr Thr Leu    290

AAC GAT GGT CTC AGT GAC AGC GAG GCT GTT GCG GTG GGT CGG TAC   1383
Asn Asp Gly Leu Ser Asp Ser Glu Ala Val Ala Val Gly Arg Tyr    305

CCT GAG GAC ACG TAC TAC AAC GGC AAC CCG TGG TTC CTG TGC ACC   1428
Pro Glu Asp Thr Tyr Tyr Asn Gly Asn Pro Trp Phe Leu Cys Thr    320

TTG GCT GCC GCA GAG CAG TTG TAC GAT GCT CTA TAC CAG TGG GAC   1473
Leu Ala Ala Ala Glu Gln Leu Tyr Asp Ala Leu Tyr Gln Trp Asp    335

AAG CAG GGG TCG TTG GAG GTC ACA GAT GTG TCG CTG GAC TTC TTC   1518
Lys Gln Gly Ser Leu Glu Val Thr Asp Val Ser Leu Asp Phe Phe    350

AAG GCA CTG TAC AGC GAT GCT GCT ACT GGC ACC TAC TCT TCG TCC   1563
Lys Ala Leu Tyr Ser Asp Ala Ala Thr Gly Thr Tyr Ser Ser Ser    365

AGT TCG ACT TAT AGT AGC ATT GTA GAT GCC GTG AAG ACT TTC GCC   1608
Ser Ser Thr Tyr Ser Ser Ile Val Asp Ala Val Lys Thr Phe Ala    380

GAT GGC TTC GTC TCT ATT GTG gtaagtctac gctagacaag cgctcatgtt   1659
Asp Gly Phe Val Ser Ile Val                                     387

gacagagggt gcgtactaac agaagtag GAA ACT CAC GCC GCA AGC AAC     1709
                               Glu Thr His Ala Ala Ser Asn      394

GGC TCC ATG TCC GAG CAA TAC GAC AAG TCT GAT GGC GAG CAG CTT   1753
Gly Ser Met Ser Glu Gln Tyr Asp Lys Ser Asp Gly Glu Gln Leu    409

TCC GCT CGC GAC CTG ACC TGG TCT TAT GCT GCT CTG CTG ACC GCC   1798
Ser Ala Arg Asp Leu Thr Trp Ser Tyr Ala Ala Leu Leu Thr Ala    424

AAC AAC CGT CGT AAC GTC GTG CCT TCC GCT TCT TGG GGC GAG ACC   1843
Asn Asn Arg Arg Asn Ser Val Val Pro Ala Ser Trp Gly Glu Thr    439
```

TCT GCC AGC AGC GTG CCC GGC ACC TGT GCG GCC ACA TCT GCC ATT 1888
Ser Ala Ser Ser Val Pro Gly Thr Cys Ala Ala Thr Ser Ala Ile 454

GGT ACC TAC AGC AGT GTG ACT GTC ACC TCG TGG CCG AGT ATC GTG 1933
Gly Thr Tyr Ser Ser Val Thr Val Thr Ser Trp Pro Ser Ile Val 469

GCT ACT GGC GGC ACC ACT ACG ACG GCT ACC CCC ACT GGA TCC GGC 1978
Ala Thr Gly Gly Thr Thr Thr Thr Ala Thr Pro Thr Gly Ser Gly 484

AGC GTG ACC TCG ACC AGC AAG ACC ACC GCG ACT GCT AGC AAG ACC 2023
Ser Val Thr Ser Thr Ser Lys Thr Thr Ala Thr Ala Ser Lys Thr 499

AGC ACC AGT ACG TCA TCA ACC TCC TGT ACC ACT CCC ACC GCC GTG 2068
Ser Thr Ser Thr Ser Ser Thr Ser Cys Thr Thr Pro Thr Ala Val 514

GCT GTG ACT TTC GAT CTG ACA GCT ACC ACC ACC TAC GGC GAG AAC 2113
Ala Val Thr Phe Asp Leu Thr Ala Thr Thr Thr Tyr Gly Glu Asn 529

ATC TAC CTG GTC GGA TCG ATC TCT CAG CTG GGT GAC TGG GAA ACC 2158
Ile Tyr Leu Val Gly Ser Ile Ser Gln Leu Gly Asp Trp Glu Thr 544

AGC GAC GGC ATA GCT CTG AGT GCT GAC AAG TAC ACT TCC AGC GAC 2203
Ser Asp Gly Ile Ala Leu Ser Ala Asp Lys Tyr Thr Ser Ser Asp 559

CCG CTC TGG TAT GTC ACT GTG ACT CTG CCG GCT GGT GAG TCG TTT 2248
Pro Leu Trp Tyr Val Thr Val Thr Leu Pro Ala Gly Glu Ser Phe 574

GAG TAC AAG TTT ATC CGC ATT GAG AGC GAT GAC TCC GTG GAG TGG 2293
Glu Tyr Lys Phe Ile Arg Ile Glu Ser Asp Asp Ser Val Glu Trp 589

GAG AGT GAT CCC AAC CGA GAA TAC ACC GTT CCT CAG GCG TGC GGA 2338
Glu Ser Asp Pro Asn Arg Glu Tyr Thr Val Pro Gln Ala Cys Gly 604

ACG TCG ACC GCG ACG GTG ACT GAC ACC TGG CGG TAGACAATCA 2381
Thr Ser Thr Ala Thr Val Thr Asp Thr Trp Arg 615

ATCCATTTCG CTATAGTTAA AGGATGGGGA TGAGGGCAAT TGGTTATATG 2431

ATCATGTATG TAGTGGGTGT GCATAATAGT AGTGAAATGG AAGCCAAGTC 2481

ATGTGATTGT AATCGACCGA CGGAATTGAG GATATCCGGA AATACAGACA 2531

CCGTGAAAGC CATGGTCTTT CCTTCGTGTA GAAGACCAGA CAGACAGTCC 2581

CTGATTTACC CTGCACAAAG CACTAGAAAA TTAGCATTCC ATCCTTCTCT 2631

GCTTGCTCTG CTGATATCAC TGTCATTCAA TGCATAGCCA TGAGCTCATC 2681

TTAGATCCAA GCACGTAATT CCATAGCCGA GGTCCACAGT GGAGCAGCAA 2731

CATTCCCCAT CATTGCTTTC CCCAGGGGCC TCCCAACGAC TAAATCAAGA 2781

| | |
|---|---|
| GTATATCTCT ACCGTCCAAT AGATCGTCTT CGCTTCAAAA TCTTTGACAA | 2831 |
| TTCCAAGAGG GTCCCCATCC ATCAAACCCA GTTCAATAAT AGCCGAGATG | 2881 |
| CATGGTGGAG TCAATTAGGC AGTATTGCTG GAATGTCGGG GCCAGTTCCG | 2931 |
| GGTGGTCATT GGCCGCCTGT GATGCCATCT GCCACTAAAT CCGATCATTG | 2981 |
| ATCCACCGCC CACGAGGGCG TCTTTGCTTT TTGCGCGGCG TCCAGGTTCA | 3031 |
| ACTCTCTCTG CAGCTCCAGT CCAACGCTGA CTGACTAGTT TACCTACTGG | 3081 |
| TCTGATCGGC TCCATCAGAG CTATGGCGTT ATCCCGTGCC GTTGCTGCGC | 3131 |
| AATCGCTATC TTGATCGCAA CCTTGAACTC ACTCTTGTTT TAATAGTGAT | 3181 |
| CTTGGTGACG GAGTGTCGGT GAGTGACAAC CAACATCGTG CAAGGGAGAT | 3231 |
| TGATACGGAA TTGTCGCTCC CATCATGATG TTCTTGCCGG CTTTGTTGGC | 3281 |
| CCTATTCGTG GGATCGATGC CCTCCTGTGC AGCAGCAGGT ACTGCTGGAT | 3331 |
| GAGGAGCCAT CGGTCTCTGC ACGCAAACCC AACTTCCTCT TCATTCTCAC | 3381 |
| GGATGATCAG GATCTCCGGA TGAATTC | 3408 |

The nucleotide sequence obtained was compared, in a computer-programmed matching operation, with the regions of known amino acid sequence of A. niger (References 1a and 1b) and A. awamori glucoamylase. The matching operation examined the nucleotide sequence in each of the six possible reading frames for codon correspondence with the given amino acid sequence. The matching operation produced nearly complete correspondence between coding regions of the glucoamylase gene and the regions of known amino acid sequence of glucoamylase from A. awamori. The amino acid sequence of one of the internal peptides of A. niger (Fig. 7 of Reference 1a) was found not to be contiguously encoded by the nucleic acid sequence (nucleotides 753-895 of Table I). An intervening sequence of 55 nucleotides was presumed to interrupt this protein coding region. The introns in the glucoamylase gene are in lower case. The amino acid sequence of the glucoamylase gene is indicated below the appropriate nucleotides and is numbered below the nucleotide sequence numbers at the right. Amino acids -24 to -1 (all capital letters) represent the signal sequence of the preglucoamylase protein.

To confirm the identification of this interrupting sequence as the intervening sequence, and to identify other intervening sequences within the glucoamylase gene, the cDNA sequences derived from glucoamylase mRNA were molecularly cloned. Double-stranded cDNA was prepared from mRNA of starch-grown A. awamori and a cDNA library was prepared in pBR322, also available from Bethesda Research Laboratories, as described above. Sixteen glucoamylase cDNA-containing plasmids were identified using pGAR1 probe; the largest plasmid, p24A2, which was deposited with the National Regional Research Laboratory in Peoria, Illinois, USA on December 7, 1983 and assigned NRRL No. B-14217, contained 1.8 kilobases of sequence derived from the 3'-end of the approximately 2.2 kilobase glucoamylase mRNA. The nucleotide sequence of the glucoamylase cDNA in p24A2 was determined and found to span the genomic sequence, shown in Table I, from nucleotide 501 through the polyadenylation site at position 2489-2491. (The precise polyadenylation site cannot be determined unambiguously due to the presence of two A residues at nucleotides 2490-2491.) Comparison of the nucleotide sequence of the molecularly cloned glucoamylase gene with that of the glucoamylase mRNA, as determined from molecularly cloned glucoamylase cDNA, and with glucoamylase amino acid sequence, has revealed the presence of four intervening sequences (introns) within the A. awamori glucoamylase gene. (The junctions of the first intervening sequence were deduced from incomplete amino acid sequence data at residues 43-49 of A. awamori glucoamylase-I.) The intervening sequences were short (ranging from 55 to 75 base pairs) and were all located within protein-encoding sequences. These sequences adjoining the intervening sequence junctions of the glucoamylase gene were compared to consensus splice junction sequences from eucaryotes in general (Reference 13) and from S. cerevisiae in particular (Reference 14). Splice junctions within the glucoamylase gene conform closely to the consensus sequences at the 5' and 3' intervening sequence termini. Sequences related to the consensus sequence TACTAACA postulated by Langford, et al. in Reference 15 to be required for splicing in S. cerevisiae are found near the 3' terminus of all glucoamylase intervening sequences.

The 5' end of the glucoamylase mRNA was determined using a synthetic oligonucleotide to prime reverse transcriptase synthesis from the mRNA template. Four major primer extension products were synthesized using the pentadecamer 5'GCGAGTAGAGATCGG3' which is complementary to sequences within the signal peptide-encoding region near the 5' end of the glucoamylase mRNA, as indicated in FIGURE 5.

The shorter band of the doublets is interpreted to represent the incompletely extended form of the longer band. To examine possible effects of RNA secondary structures on this pattern, primer extension was preferred at 42 and 50 °C. The products of primer extension at 42 °C (lane 1) and 50 °C (lane 2) are displayed on a sequencing gel described in Reference 16 in parallel with m13/dideoxynucleotide sequencing reactions of this region, using the identical pentadecamer primer. The sequence presented in FIGURE 5 represents the glucoamylase mRNA sequence and is complementary to that read from the sequencing reactions shown. The pattern of primer extension was unchanged, supporting the conclusion that four distinct 5' termini exist within the population of glucoamylase mRNA. Primer extension reactions performed in the presence of dideoxynucleotides confirmed the colinearity of genomic and mRNA sequences in this region. The primer extension products map to T residues, at positions -71, -66, -59, and -52 from the site of translation initiation, and are indicated in Table I. To the extent that reverse transcriptase is able to copy the extreme terminal nucleotide(s) of the mRNA, the 5' termini of the glucoamylase mRNAs are localized to these four regions. DNA sequences 5' of the region of transcription initiation were found to contain sequences homologous to consensus sequences previously shown to be involved in transcription initiation by RNA polymerase II.

Table IIA illustrates the nucleotide sequence encoding the mature glucoamylase polypeptide.

TABLE IIA

GCG ACC TTG GAT TCA TGG TTG AGC AAC GAA GCG ACC GTG GCT CGT
ACT GCC ATC CTG AAT AAC ATC GGG GCG GAC GGT GCT TGG GTG TCG
GGC GCG GAC TCT GGC ATT GTC GTT GCT AGT CCC AGC ACG GAT AAC
CCG GAC TAC TTC TAC ACC TGG ACT CGC GAC TCT GGT CTC GTC CTC
AAG ACC CTC GTC GAT CTC TTC CGA AAT GGA GAT ACC AGT CTC CTC
TCC ACC ATT GAG AAC TAC ATC TCC GCC CAG GCA ATT GTC CAG GGT
ATC AGT AAC CCC TCT GGT GAT CTG TCC AGC GGC GCT GGT CTC GGT GAA
CCC AAG TTC AAT GTC GAT GAG ACT GCC TAC ACT GGT TCT TGG GGA
CGG CCG CAG CGA GAT GGT CCG GCT CTG AGA GCA ACT GCT ATG ATC
GGC TTC GGG CAA TGG CTG CTT GAC AAT GGC TAC ACC AGC ACC GCA
ACG GAC ATT GTT TGG CCC CTC GTT AGG AAC GAC CTG TCG TAT GTG
GCT CAA TAC TGG AAC CAG ACA GGA TAT GAT CTC TGG GAA GAA GTC
AAT GGC TCG TCT TTC TTT ACG ATT GCT GTG CAA CAC CGC GCC CTT
GTC GAA GGT AGT GCC TTC GCG ACG GCC GTC GGC TCG TCC TGC TCC
TGG TGT GAT TCT CAG GCA CCC GAA ATT CTC TGC TAC CTG CAG TCC
TTC TGG ACC GGC AGC TTC ATT CTG GCC AAC TTC GAT AGC AGC CGT
TCC GGC AAG GAC GCA AAC ACC CTC CTG GGA AGC ATC CAC ACC TTT
GAT CCT GAG GCC GCA TGC GAC GAC TCC ACC TTC CAG CCC TGC TCC
CCG CGC GCG CTC GCC AAC CAC AAG GAG GTT GTA GAC TCT TTC CGC
TCA ATC TAT ACC CTC AAC GAT GGT CTC AGT GAC AGC GAG GCT GTT
GCG GTG GGT CGG TAC CCT GAG GAC ACG TAC TAC AAC GGC AAC CCG
TGG TTC CTG TGC ACC TTG GCT GCC GCA GAG CAG TTG TAC GAT GCT
CTA TAC CAG TGG GAC AAG CAG GGG TCG TTG GAG GTC ACA GAT GTG
TCG CTG GAC TTC TTC AAG GCA CTG TAC AGC GAT GCT GCT ACT GGC
ACC TAC TCT TCG TCC AGT TCG ACT TAT AGT AGC ATT GTA GAT GCC
GTG AAG ACT TTC GCC GAT GGC TTC GTC TCT ATT GTG GAA ACT CAC

GCC GCA AGC AAC GGC TCC ATG TCC GAG CAA TAC GAC AAG TCT GAT

GGC GAG CAG CTT TCC GCT CGC GAC CTG ACC TGG TCT TAT GCT GCT

CTG CTG ACC GCC AAC AAC CGT CGT AAC GTC GTG CCT TCC GCT TCT

TGG GGC GAG ACC TCT GCC AGC AGC GTG CCC GGC ACC TGT GCG GCC

ACA TCT GCC ATT GGT ACC TAC AGC AGT GTG ACT GTC ACC TCG TGG

CCG AGT ATC GTG GCT ACT GGC GGC ACC ACT ACG ACG GCT ACC CCC

ACT GGA TCC GGC AGC GTG ACC TCG ACC AGC AAG ACC ACC GCG ACT

GCT AGC AAG ACC AGC ACC AGT ACG TCA TCA ACC TCC TGT ACC ACT

CCC ACC GCC GTG GCT GTG ACT TTC GAT CTG ACA GCT ACC ACC ACC

TAC GGC GAG AAC ATC TAC CTG GTC GGA TCG ATC TCT CAG CTG GGT

GAC TGG GAA ACC AGC GAC GGC ATA GCT CTG AGT GCT GAC AAG TAC

ACT TCC AGC GAC CCG CTC TGG TAT GTC ACT GTG ACT CTG CCG GCT

GGT GAG TCG TTT GAG TAC AAG TTT ATC CGC ATT GAG AGC GAT GAC

TCC GTG GAG TGG GAG AGT GAT CCC AAC CGA GAA TAC ACC GTT CCT

CAG GCG TGC GGA ACG TCG ACC GCG ACG GTG ACT GAC ACC TGG CGG

Nucleotides 206 to 277 encode the signal sequence for the A. awamori glucoamylase. As used in the specification and claims, the term "signal sequence" refers generally to a sequence of amino acids which are responsible for initiating export of a protein chain. A signal sequence, once having initiated export of a growing protein chain, is cleaved from the mature protein at a specific site. The term also includes leader sequences or leader peptides. The preferred signal sequence herein is the deduced signal sequence from the A. awamori glucoamylase gene given in Table IIB.

TABLE IIB

MET SER PHE ARG SER LEU LEU ALA LEU SER GLY LEU VAL CYS THR

GLY LEU ALA ASN VAL ILE SER LYS ARG

EXAMPLE 2

Expression of Glucoamylase Gene in Yeast

A. Construction of HindIII Cassette of Genomic Glucoamylase Gene

A method for expressing genes at high levels in yeast involves constructing vectors which contain the yeast enolase I promoter and terminator regions (Reference 16). The enolase segments were previously engineered so that the promoter and terminator were separated by a unique HindIII site.

Plasmid pAC1 (10.67 kilobase) is an E. coli/yeast shuttle vector, capable of autonomous replication in both E. coli and yeast strains. The plasmid confers resistance in E. coli and related species to the β-lactam antibiotic ampicillin and related compounds as a result of synthesis of the TEM type I β-lactamase. Further, the plasmid carries the yeast LEU2 gene which is expressed in both E. coli and S. cerevisiae strains. Thus, the presence of the plasmid in either E. coli or S. cerevisiae strains reverses a leucine growth requirement resulting from loss of β-isopropylmalate dehydrogenase activity.

Plasmid pAC1 is comprised of the following DNA segments. Numbering starts at the EcoRI site of the enolase I promoter fragment and proceeds in a clockwise direction. Coordinates 0 to 725 comprise a 725 base pair EcoRI to HindIII DNA fragment derived from a similar fragment in the plasmid p eno 46 (Reference 16), containing DNA from the 5' untranslated region of the S. cerevisiae EnoI gene. This fragment has been modified in the region just prior to the initiation codon (ATG) of the enolase gene in order to create a HindIII site. Specifically, the sequence was changed from CACTAAATCAAAATG to CACGGTCGAGCAAGCTT(ATG). Coordinates 726 to 2281 comprise the 1.55 kilobase HindIII to BglII DNA fragment from the 3' untranslated region of the S. cerevisiae EnoI gene and was originally obtained from the plasmid peno 46 (Reference 16). Coordinates 2282 to 2557 comprise a 275 basepair DNA fragment from the plasmid pBR322 (Reference 16a) between the BamHI and SalI recognition sites (pBR322 coordinates 375 to 650). Coordinates 2558 to 4773 comprise the 2.22 kilobase XhoI to SalI DNA fragment from S. cerevisiae that encodes the LEU2 gene product, β-isopropylmalate dehydrogenase. The plasmid YEp13 (Reference 16b) provided a convenient source for the desired 2215 basepair DNA fragment. Coordinates 4474 to 8528 comprise a 3.75 kilobase DNA fragment which permits autonomous replication of the plasmid AC1 in yeast strains. This region encodes a portion of the yeast 2μ plasmid and was derived from the plasmid pDB248 (Reference 16c). Digestion of plasmid pDB248 with the enzymes EcoRI and SalI liberated the desired 3.75 kilobase DNA fragment incorporated in plasmid AC1. Coordinates 8529 to 10672 comprise DNA sequences which permit autonomous replication in E. coli host strains and confer ampicillin resistance. The desired 2143 basepair DNA fragment was obtained from E. coli plasmid pBR322 as a Tth111I to EcoRI DNA fragment (pBR322 coordinates 2218 and 4360, respectively). A sample of E. coli K12 strain MM294 transformed with pAC1 was deposited in the American Type Culture Collection on December 2, 1983 and has been assigned ATCC No. 39,532.

The glucoamylase gene, while not having a convenient restriction site closely preceding its initiation codon (ATG) useful for cloning into vectors, can have a single base pair change 32 base pairs upstream from the ATG so as to create a unique HindIII site, allowing use of the enolase promoter for initiation of transcription. Site-specific mutagenesis was used to obtain the desired mutation. A hexadecamer oligonucleotide which is complementary to the region surrounding the desired HindIII site and which contains the appropriate mismatch was used to prime DNA synthesis on a single-stranded M13 template of the glucoamylase gene. The sequence of the primer employed was : GAGCCGAAGCTTCATC, with the mismatches underlined. A second mismatch was incorporated into the primer to aid in the screening for correct clones by hybridizing candidate plaques with the same oligonucleotide used for the primer extension, after the latter had been radioactively labeled.

One picomole of a single stranded DNA phage, M13mp9 containing a 2.3 kilobase glucoamylase gene fragment (from EcoRI to SalI), was annealed to 10 picomoles of the primer in a 15 μl reaction mix which also contained 20 mM Tris pH 7.9, 20 mM $MgCl_2$, 100 nM NaCl, and 20 mM β-mercaptoethanol. The mixture was heated to 67° C, incubated at 37° C for 30 minutes, then placed on ice.

To the above annealing mixture 1 μl of each deoxynucleotide triphosphate at 10 mM was added, to a final concentration of 500 μM. Five units of E. coli Klenow fragment of DNA polymerase I (0.5 μl) was then added and the extension reaction was left on ice for 30 minutes. Starting on ice minimizes 3' -5' exonuclease digestion of the primer and subsequent mismatch correction. After 30 minutes on ice, the reaction was continued at 37° C for 2 hours, then inactivated by heating at 67° C for 10 minutes.

Note that the primer was not kinased and no ligase was used in contrast to other published methods. JM103 competent cells were transformed with 1 μl of the reaction and either 5 μl or 50 μl were plated.

The hexadecamer used for priming was kinased with labeled $^{32}$P-ATP to a specific activity of 3 x $10^7$ cpm/μg. Nitrocellulose filters were used to bind phage DNA from the plaques by direct lifting, and these filters were denatured, neutralized and washed. After baking for 2 hours at 80° C, the filters were prehybridized for 3 hours at 45° C in 25 ml of a solution of 9 M NaCl and 0.9 M sodium citrate, sodium dodecylsulfate, 50 ml of a solution of 0.5 g bovine serum albumin, 0.5 g Ficoll 400 (a carbohydrate polymer) and 0.5 g polyvinylpyrrolidine, and 50 μg/ml yeast RNA. After prehybridization, 1.5 x $10^5$ cpm/ml of kinased primer was added, and hybridization continued overnight at 45° C. The next day, filters were washed 2 times, 5 minutes each in a solution of 9 M NaCl and 0.9 M sodium citrate at roughly 5° C (to remove non-specifically bound counts), then once at 45° C for 5 minutes (to remove probe hybridized to non-mutant phage DNA). Filters were air dried, put on Kodak XAR (high speed) film with an intensifying screen and exposed overnight at -70° C.

One mutant clone among several thousand plaques was discovered in the first round of screening.

Subsequent restriction enzyme digests of this clone confirmed the introduction of the HindIII site in front of the glucoamylase gene.

In the next step a HindIII site was created at the 3' end of the glucoamylase gene. A clone with the engineered HindIII site near the 5' end of the gene was cut with NcoI, its sticky ends were converted to blunt ends by enzymatic repair using Klenow fragment of E. coli DNA polymerase-I, and it was cut with EcoRI. FIGURE 7 illustrates a restriction map of this region. This method produced a fragment containing the glucoamylase gene and having an EcoRI sticky end before the 5' end of the gene and a blunt end after the 3' end of the gene. This fragment was cloned into a polylinker region of plasmid pUC8, available from Bethesda Research Laboratories, to place a HindIII site within 20 nucleotides of the 3' end of the fragment so as to produce a HindIII cassette.

B. Construction of Full-Length cDNA Clone of Glucoamylase Gene Lacking Introns

The longest cDNA clone produced and isolated which had regions homologous to the genomic clone of the glucoamylase gene, p24A2, corresponds in sequence to the genomic clone from nucleotides 501 to 2490, minus the nucleotides corresponding to introns indicated in lower case in Table I. This clone is still several hundred nucleotides shorter than necessary for a full-length cDNA clone. The construction of a full-length cDNA copy of the gene was accomplished in several steps. The genomic clone with the HindIII site near the 5' end of the gene was cut with EcoRI and AvaII and this fragment was purified. The longest cDNA clone described above was digested with AvaII and PstI, and the small AvaII to PstI fragment was purified. The phage vector M13mp11, available from P-L Biochemicals, 1037 W. McKinley Ave., Milwaukee, WI 53205, was digested with EcoRI arid PstI, and the large vector fragment was purified from the small polylinker fragment. These three fragments were ligated together to generate a M13mp11 vector containing the EcoRI and PstI region of the genomic clone, but now missing the second intron.

The longest cDNA clone was then cut with PstI using conditions supplied by the manufacturer of the restriction enzyme and the large PstI fragment was isolated. The M13mp11 vector described above was cut with PstI, and the large PstI fragment from the cDNA clone was ligated into this site. The clones generated from this ligation were screened to identify the clone with the PstI fragment inserted in the correct orientation. The clone isolated from this step had the genomic sequence from EcoRI to AvaII (containing the first intron and the new 5' HindIII site) and the cDNA sequence from AvaII to the PstI site beyond the poly-A tail region. The remaining intron at the 5' end of the gene was removed by site-directed mutagenesis using a nonacosamer oligonucleotide to span the intron region. The nonacosamer, which had homology to 15 base pairs on the 5' side of the intron and 14 base pairs on the 3' side, had the sequence:

**5' CGGATAACCCGGACTACTTCTACACCTGG 3'**

In the procedure for conducting site-directed mutagenesis, one picomole of a single-stranded DNA phage derivative designated as M13mp9 (which is commercially available), containing a 2.3 kilobase glucoamylase gene fragment (from EcoRI to SalI), was annealed to 10 picomoles of primer in 15 µl containing 6 mm of tris(hydroxymethyl)aminomethane (hereinafter Tris) at pH 7.9, 6 mm $MgCl_2$ and 100 mM NaCl. The mixture was heated to 67° C, incubated at 37° C for 30 minutes, and then placed on ice. At this temperature, either half of the nonacosomer can anneal to its complement on the template without the other, allowing the proper loop to be formed.

To the above annealing mixture 1 µl of each deoxynucleotide triphosphate at 10 mM was added, to a final concentration of 500 µM. Five units of E. coli Klenow fragment of DNA polymerase I (0.5 µl) was then added and the extension reaction was left on ice for 30 minutes to minimize 3'-5' exonuclease digestion of the primer. After 30 minutes on ice, the reaction was continued at 37° C for 2 hours, and then inactivated by heating at 67° C for 10 minutes.

In the procedure employed herein the primer was not kinased and no ligase was employed in contrast to other published methods. JM 103 competent cells were transformed with 1 µl of the reaction and either 5 µl or 50 µl were plated. (JM103 is an E. coli strain distributed by Bethesda Research Laboratories, Inc., Gaithersburg, MD 20877.)

The nonacosamer used for priming was kinased with labeled $^{32}$P-ATP to a specific activity of 3 x $10^7$ cpm/µg. Nitrocellulose filters were employed to bind phage DNA from the plaques by direct lifting, and these filters were denatured, neutralized and washed. After baking for 2 hours at 80° C, the filters were prehybridized for 3 hours at 55° C in 25 ml of a solution of 9 M NaCl, 0.9 M sodium citrate, 0.1% sodium dodecyl sulfate, 50 ml of a solution containing 0.5 g bovine serum albumin, 0.5 g Ficoll 400 (which is a carbohydrate polymer obtainable from Pharmacia Fine Chemicals) and 0.5 g polyvinylpyrrolidone, and finally 50 µg/ml yeast RNA. After prehybridization, 1.5 x $10^5$ cpm/ml of kinased primer was added, and hybridization was continued overnight at 55° C.

The next day, the filters were washed two times for five minutes each in a solution of 9 M NaCl and 0.9 M sodium citrate at roughly 5° C (to remove non-specifically bound counts), and then once at 55° C for five minutes (to remove probe hybridized to non-mutant phage DNA). Filters were air-dried and placed on Kodak XAR (high speed) film with an intensifying screen and exposed overnight at -70° C.

The frequency of positives recovered was about 4%. Positive candidate plaques were further examined by preparing mini-preps and digesting them to see if a size reduction occurred due to removal of the 75 base pair intron. Sequencing of one of the positives revealed that the intron had been precisely removed.

In the final step this plasmid vector was digested with EcoRI and BamHI, and the fragment was purified and used to replace the EcoRI to BamHI fragment in the genomic HindIII cassette vector described under section A above. The result is a cDNA HindIII cassette which will have the normal polyadenylation signal at the 3' end of the clone but lacks all four introns.

C. Yeast Strains Transformed with Yeast Expression Vector

The intron-containing HindIII cassette of the genomic glucoamylase gene as described in section A above was excised and inserted into a yeast expression vector plasmid pAC1 to produce a plasmid designated as pGAC9, the map of which is presented in FIG. 7. A sample of E. coli K12 strain MM294 transformed with pGC21 was deposited in the NRRL on December 7, 1983 and has been assigned NRRL No. B-14215. A sample of E. coli K12 strain MM294 transformed with pAC1 was deposited in the American Type Culture Collection on December 2, 1983 and has been assigned ATCC No. 39,532. The cassette of full-length cDNA clone lacking introns as described in section B above was similarly excised and inserted into the vector pAC1 to produce a plasmid designated as pGC21, the map of which is presented in FIG. 8.

Plasmid DNAs pGC21 and pGAC9 were amplified in E. coli, purified on a cesium chloride gradient and used to transform two strains of yeast: yeast strain C468, which is a haploid Saccharomyces cerevisiae with auxotrophic markers for leucine and histidine, and yeast strain H18, which is a haploid S. cerevisiae with auxotrophic markers for leucine and histidine, which lacks the repressor for the glucoamylase gene of Saccharomyces diastaticus. $Leu^+$ transformants were screened for expression of the Aspergillus awamori glucoamylase gene. H18 was deposited in the National Regional Research Laboratory in Peoria, Illinois, USA on December 7, 1983 and has been assigned NRRL Number Y-12842.

Yeast strains which were transformed with the yeast expression vectors pGC21 and pGAC9 were compared with the same strains transformed with the parent plasmid pAC1 as a control for growth on various starches in liquid and on solid media. Three types of starch were used: "washed" starch (a soluble starch washed three times with 70% ethanol to remove sugars and short chain carbohydrates), cassava starch, and soluble potato (Baker's) starch. Yeasts transformed with any of the three plasmids grew on the three starches; however, the cDNA clones (pGAC9) always showed better growth than the other clones, both in liquid and on solid media. When Baker's starch, which is the most highly polymerized of the three starches, was used in solid media at a concentration of 2% (w·v), the plates were turbid. These plates were spread with yeast from both strains carrying the parent plasmid, the genomic clone or the cDNA clone, and with yeast strain Saccharomyces diastaticus, having NRRL Deposit No. Y-2044, which expresses a yeast glucoamylase. The plates are shown in FIG. 9. The strains carrying the cDNA clone (pGAC9) were able to clear the starch around the growth zone, indicating that they could degrade the starch completely. In contrast, the S. diastaticus strain and the yeast strains transformed with either the parent plasmid pAC1 or the genomic clone pGC21 were unable to clear the starch from around the growth area. The clearing of the highly polymerized starch exhibited by pGAC9-containing strains indicates the functional expression of the A. awamori glucoamylase gene that has both alpha 1-4 and alpha 1-6 amylase activity.

In another test for glucoamylase expression, yeast cells carrying the control plasmid, pAC1, or the cDNA clone, pGAC9, were grown in a washed starch liquid medium. The cells were harvested and lysed by ten cycles of freeze, thaw, and vortexing with glass beads. Each cell lysate, containing intracellular proteins, was electrophoresed on a 7% acrylamide gel containing 0.1% sodium dodecyl sulfate (SDS) and 7.6 M urea and transferred to cellulose paper activated with cyanogen bromide. After the proteins were transferred, the paper was first probed with antiserum from a rabbit immunized against A. awamori glucoamylase and then with radioactively labeled Staph A protein that binds to antibody molecules. After unbound radioactivity was washed off, the paper was dried and exposed to X-ray film. This technique, which is called a "Western" and is described in Reference 17, can be performed with antiserum or purified antibody.

Protein that reacts with glucoamylase antisera was detected in the lysates from the pGAC9 cDNA clones but not in the pAC1 controls.

The expression of the A. awamori glucoamylase gene was also tested directly by the ability of a yeast containing such a gene to grow on an otherwise non-utilizable carbon source. For yeast strains C468 and H18, this growth test was accomplished using maltose as the carbon source, because both of these strains carry a mutation (mal) blocking the utilization of maltose as a carbon source. The ability of strains C468 and H18 containing the control plasmid pAC1 or the cDNA plasmid pGAC9 to grow on maltose and glucose as a carbon source is indicated in Table III. The glucose plates contained histidine while the maltose plates contained both histidine and leucine supplementation. From this table it can be seen that the presence of the glucoamylase gene on the plasmid allows C468 to grow slowly on maltose and H18 to grow slightly better than the control.

These tests indicate that the presence of the glucoamylase gene complements the mal mutation in C468 and facilitates direct selection experiments where the growth of the yeast is solely dependent on proper and adequate functioning of the A. awamori glucoamylase gene.

All of these experiments demonstrate that yeast strain C468 containing the plasmid pGAC9 is most superior in expressing the glucoamylase gene A sample of yeast strain C468 transformed with pGAC9 was deposited with the American Type Culture Collection on November 17, 1983 and has been assigned the ATCC Deposit No. 20,690.

Table III

| Yeast Strain | Plasmid | Growth Response of Strain** Carbon Source: Glucose day2 | Glucose day4 | Glucose day6 | Maltose day2 | Maltose day4 | Maltose day6 | Maltose day10 | Maltose day13 |
|---|---|---|---|---|---|---|---|---|---|
| C468 | pAC1* | ± | + | + | 0 | 0 | 0 | 0 | 0 |
| C468 | pGAC9 | ± | + | + | 0 | 0 | m | ± | + |
| H18 | pAC1* | ± | + | + | 0 | 0 | 0 | 0 | 0 |
| H18 | pGAC9 | ± | + | + | 0 | 0 | 0 | 0 | m |

*Control
**0 = no visible colonies
m = minute colonies < 0.3 mm
± = small colonies < 1 mm
+ = normal colonies 2-3 mm

D. 1. Characterization of Glucoamylase Activity in Yeast Cultures

Standing cultures of yeast strain C468 containing pAC1 or containing pGAC9 prepared as described above were grown in minimal media with glucose or washed Difco soluble starch as the carbon sources. The cultures were harvested, after 5 days for the glucose cultures and after 7 days for the starch cultures, and cell-free supernatants were prepared by centrifugation. These supernatants were concentrated 10-20 fold using an Amicon concentrator with a PM10 membrane. Glucoamylase assays were negative for the supernatants from the glucose- and starch-grown cultures of yeast strain C468 containing pAC1 plasmid. In contrast, cells containing the control plasmid pGAC9 secreted approximately six units of glucoamylase activity per liter. (For a definition of a unit of glucoamylase activity, see the legend to Table IV).

Glucoamylase production in aerobic shake-flask cultures of yeast strain C468 containing pGAC9 plasmid was then assayed. After two days of incubation at 30° C and agitation at 250 rpm, the culture of C468 yeast strain containing pGAC9 had consumed all of the glucose and was in stationary phase. The culture had achieved a cell density of approximately 2 g/liter dry weight. A glucoamylase assay on the unconcentrated supernatant indicated that approximately 47 units of activity per liter of supernatant was produced.

2. Location of Glucoamylase Activity in Cultures of Transformed Yeast Cells

The experiment given below was used to resolve whether the majority of the glucoamylase activity is found in the culture medium or inside the cell.

Strains C468-pGAC9 and C468-pAC1 were grown in 500 ml of medium containing 1.45 g of Difco Yeast nitrogen base (Difco Laboratories, Detroit, MI 48232), 5.2 g of ammonium sulfate and 2% glucose per liter to a cell density of 2-3 x $10^7$ cells per ml. The cultures were centrifuged at 4° C and the supernatants and cell pellets were processed separately. The supernatant samples were filtered through a 0.45 μ filter and then concentrated 15 to 20X using an Amicon stirred cell with a PM-10 membrane. The cell pellet was washed once in 1 M Sorbitol 0.1 M phosphate buffer pH 7.5 and then the packed cell volume was determined by centrifuging at approximately 1000xg for 5 minutes in a conical graduated centrifuge tube. Each ml of packed cells was resuspended to 1.5 ml in 1.0 M Sorbitol-0.1 M phosphate buffer at pH 7.5 and and equal volume of Zymolyase 5000 (Miles Laboratory, Elkhart IN 46515) was added. The cells were gently mixed at room temperature for 1 hr and then centrifuged at 500xg to recover the protoplasts. The supernatant, representing the protein that was present between the cell wall and the inner membrane, was put on ice for later processing. The space between the cell membrane and wall in yeast is referred to as the interstitial space and this protoplast supernatant sample will be referred to as the interstitial sample in the following text. The protoplasts were resuspended in 1 M Sorbitol-0.1 M $KPO_4$ buffer-10 mM $NaN_3$ and washed 1x by centrifuging at 500xg. The pellet was resuspended in 5 ml 1 M Sorbitol-0.1 M $KPO_4$ at pH 7.5-10 mM $NaN_3$ and 1 ml was used to assay the glucoamylase activity present in the intact, azide-treated protoplasts. To the remaining 4 ml of protoplast 4 ml of 50 mM Tris at pH 7.4-10 mM EDTA was added along with 6 g of sterile glass beads (0.45-0.5 mm B. Braun) and the mixture was vortexed vigorously for 20 seconds, cooled on ice and this procedure was repeated until microscopic observation revealed membrane ghosts or particles but few or no intact protoplasts. Sterile 2 M sucrose was added slowly with a pasteur pipette inserted to the bottom of the tube and the lysate was floated out of the glass beads. The lysate was removed to a new tube and centrifuged along with the interstitial sample at approximately 20,000xg for 30 min at 4° C. The supernatant from the broken protoplasts was designated the intracellular sample and the pellets from the interstitial sample and the broken protoplast sample were combined to make the membrane sample. Thus the yeast culture has been fractionated into five samples: the extracellular or supernatant sample, the interstitial, membrane associated and intracellular samples, as well as a sample containing intact azide-treated protoplasts.

The culture samples were analyzed for glucoamylase activity utilizing the peroxidase-glucose oxidase (PGO)/o-dianisidine (ODAD) assay (Sigma Kit #510) which detects glucose released from soluble starch by the glucoamylase. The assay can be affected by other enzymes present which utilize glucose or by glucose present in the samples. Each PGO-ODAD Assay mix was tested with known quantities of glucose (generally a dilution series from 0 to 550 nanomoles) and a standard curve was constructed. One glucoamylase unit is defined as the amount of glucoamylase which releases one μmole of glucose per minute from washed soluble starch at 37° C.

Samples were reacted with washed soluble starch on the day they were prepared, then boiled and frozen at -20° C for later glucose assay. A portion of each fresh sample was precipitated by addition of 3 volumes of cold 95% ethanol, then allowed to stand overnight and the precipitate was collected by centrifugation at 2000xg for 5 min at 4° C. The supernatant sample required a second centrifugation to recover small flocs which remained suspended in the ethanol supernatant. The pellets were dried and then resuspended in 50 mM Tris at pH 7.4-10 mM EDTA to one half their original volume, except the supernatant sample which was resuspended to one twentieth its original volume. These ethanol-precipitated samples were reacted with washed soluble starch and then boiled and frozen -20° C for assay with the fresh samples.

Intact azide-treated protoplasts were assayed in a reaction mix containing 1 M Sorbitol-0.5% washed starch and 200 μl of protoplasts. These mixes were incubated at 37° C for 30 min, then centrifuged at 500xg and the supernatant was filtered, then boiled and assayed or stored at -20° C. These assays revealed that the reaction mix contained some residual glucose and that the protoplasts reduced the amount of glucose in the mix during incubation. When lysed protoplasts were incubated in the same mix, more glucose was utilized than when the protoplasts were intact. Values for the glucoamylase plasmid carrying strain were similar to those for the strain carrying the same plasmid without the glucoamylase DNA insert, implying that little, if any, glucoamylase activity is associated with the membrane.

The fresh fractionated samples were assayed and the intra-cellular samples were found to have residual glucose levels that were too high for the assay. Membrane-associated and interstitial samples from pGAC9- and pAC1-transformed cells both failed to produce detectable levels of glucose from soluble starch. The supernatant sample from pGAC9-transformed yeast demonstrated glucoamylase activity of about 22 units/liter, while the sample from pAC1-transformed yeast showed no glucoamylase activity. Ethanol-precipitated samples from the pAC1-transformed yeast showed negligible (less than or equal to 0.08 units/liter) or no glucoamylase activity. Ethanol-precipitated samples from yeast transformed with pGAC9 all demonstrated glucoamylase activity of 0.15 units per liter or higher. The supernatant sample contained over 90% of the total glucoamylase activity and the intracellular, membrane associated and interstitial samples contained from 1 to 4% of the total activity depending on the sample. Therefore, most of the glucoamylase enzyme is secreted into the extracellular medium.

E. Production of Recombinant Glucoamylase from Yeast in a 10 Liter Fermentor

To produce sufficient glucoamylase for characterization, a 10-liter fermentation of C468 yeast strain containing pGAC9 in minimal media with glucose as the sole carbon source was set up. A 100-ml seed culture was grown in minimal media to an optical density at 280 nm ($OD_{280}$) of 6 and added to the fermentor. The fermentor was run as an aerobic batch fermentation until it reached an $OD_{280}$ of 10, and then a glucose feed was begun. The glucose feed was continued to an $OD_{280}$ of approximately 30 and then stopped, allowing the residual glucose to be consumed. Total fermentation time was approximately 32 hours. The final cell density was approximately 10 g/liter dry weight. Diluted samples of the unconcentrated fermentor supernatant were assayed for glucoamylase activity, with the assay data given in Table IV. The supernatant was concentrated 15-fold using an Amicon Hollow Fiber Concentration unit with a 10,000 molecular weight size exclusion.

Table IV

Recombinant Glucoamylase Purification

| Sample | Glucoamylase Activity* (units) | Volume (ml) | Protein** (mg) | Percent Recovery | Specific Activity (units/mg) |
|---|---|---|---|---|---|
| Fermentor Supernatant | 3146 | 10,000 | -- | 100 | -- |
| Concentrated Supernatant | 1605 | 660 | 219 | 51 | 7.3 |
| DEAE-Sepharose Column | 2300 | 160 | 173 | 73 | 13.3 |

* One unit of glucoamylase activity is the release of 1 μmole glucose/minute from washed Difco soluble starch in 0.1 M citrate buffer, pH 5.0, at 37°C.

** The protein concentration of the concentrated supernatant was determined using a BioRad protein assay kit. The protein concentration from the DEAE-Sepharose column was estimated by integration of area under the $OD_{280}$ peak (1 $OD_{280}$ units = 1 mg/ml protein).

The concentrated fermentor supernatant was adjusted to 50 mM phosphate, pH 7.5, by adding concentrated buffer thereto and was loaded on a DEAE Sepharose® (CL-6B) column. The column was eluted with a pH gradient (starting pH 75, final pH 3.0). The elution profile is shown in FIG. 10. Various samples from the column were analyzed by SDS-urea polyacrylamide gel electrophoresis. A photograph of the gel stained with BioRad silver stain showed that the concentrated fermentor supernatant contained only a few proteins, demonstrating that the glucoamylase was secreted into the media and not released by cell lysis. A comparison of a sample from this concentrated fermentor supernatant with an equal volume of the peak fraction of glucoamylase activity indicated a considerable increase in the purity of the protein. Estimates indicated that 20-30% of the supernatant protein was glucoamylase and the peak fraction was approximately 80% glucoamylase. The recombinant glucoamylase migrated with a mobility slightly slower than the A. awamori glucoamylase, indicating that the glucoamylase produced in the transformed yeast was also glycosylated.

An assay on the peak column fraction of glucoamylase activity indicated that the recombinant glucoamylase has a specific activity comparable to native A. awamori glucoamylase, namely 25-50 units/mg.

Experiments prove that the recombinant glucoamylase produced by yeast C468/pGAC9 is glycosylated. Duplicate samples of A. awamori glucoamylase-I and glucoamylase-II and the recombinant glucoamylase were electrophoresed in a 10% polyacrylamide-SDS gel using standard procedures. After electrophoresis, the gel was split and lanes 1-4 were stained for protein with a Coomassie Blue stain and lanes 5-7 were stained for carbohydrate with Periodic Acid Schiff's stain. Details of these procedures are found in Reference 18. A comparison of glucoamylase-I (lanes 2 and 5), glucoamylase-II (lanes 3 and 6) and the recombinant glucoamylase (lanes 4 and 7) is shown in FIGURE 11. Since the bands corresponding to these proteins also stain with the carbohydrate stain, this demonstrates that the recombinant glucoamylase is glycosylated by the yeast.

EXAMPLE 3

Production of Alcohol from Transformed Yeast

Yeast strain C468 containing pGAC9, and the control C468 yeast strain containing pAC1 were inoculated into 50 ml of the following medium:

| | |
|---|---|
| succinic acid | 11.81 g |
| $H_3PO_4$ | 0.58 g |
| $H_2SO_4$ | 0.31 g |
| KCl | 0.37 g |
| NaCl | 58.4 mg |
| $MgCl_2 \cdot 6H_2O$ | 0.2 g |
| $MnSO_4 \cdot H_2O$ | 1.7 mg |
| $CuSO_4 \cdot 5H_2O$ | 0.25 mg |
| $ZnSO_4 \cdot 7H_2O$ | 1.44 mg |
| $CoCl_2 \cdot 6H_2O$ | 1.19 mg |
| $Na_2MoO_4 \cdot 2H_2O$ | 1.21 mg |
| $H_3BO_3$ | 3.09 mg |
| $CaCl_2 \cdot 2H_2O$ | 14.7 mg |
| $FeSO_4 \cdot 7H_2O$ | 11.1 mg |
| histidine | 40 mg |
| washed soluble starch* | 100 g |
| add water in quantities sufficient to 1 liter | |

*The starch was washed three times in 70% ethanol to remove low molecular weight carbohydrates. The precipitate was then dried, but some ethanol and water may have remained.

Fermentation was carried out in 250 ml flasks which were equipped with air restrictors to restrict the flow of oxygen into the flask. The flasks were incubated at 32°C and shaken at 200 rpm for 7 days.

The ethanol content of each flask was evaluated using gas chromatography. The C468/pGAC9 culture contained 23.4 g/l ethanol while the control C468/pAC1 culture contained 4.5 g/l ethanol. The results show that the production of glucoamylase by the C468/pGAC9 culture enabled the strain to convert the soluble starch into glucose and then to ferment the glucose to ethanol.

EXAMPLE 4

Expression of the Glucoamylase Gene in E. coli

In order to express the glucoamylase gene in E. coli, a modification was made to the 5' untranslated region in order to make the DNA sequence more compatible with transcription and translation in E. coli. Specifically, 27 base pairs between the HindIII site which was constructed 32 base pairs upstream from the ATG initiation codon (see Example 2) and the ATG codon were deleted by oligonucleotide mutagenesis using the procedure described in Example 2B for removal of an intron. The oligonucleotide, which had homology to 12 base pairs on the 5' side of the region to be deleted and 11 base pairs on the 3' side, had the sequence:

5' GAGCCGAAGCTTTATGTCGTTCCG 3'

Except for this deletion, the final HindIII cassette was identical to that constructed for the yeast expression vector in Example 2.

E. coli expression vector ptrp3 was constructed by replacing the EcoRI to ClaI region of pBR322 (coordinates -3 to 28, see Reference 16a) with an EcoRI to ClaI fragment containing the E. coli tryptophan promoter and ribosome binding site. The nucleotide sequence of this region is shown in Table V; the EcoRI. ClaI and HindIII sites have been identified in Table V.

Table V

GAA TTC CGA CAT CAT AAC GGT TCT GGC AAA TAT TCT GAA ATG
EcoRI
AGC TGT TGA CAA TTA ATC

ATC GAA CTA GTT AAC TAG TAC GCA AGT TCA CGT AAA AAG GGT
ATC GAT AAG CTT
ClaI HindIII

The HindIII cassette of the glucoamylase gene, described above in this example, was cloned into the HindIII site of ptrp3. Transformants were screened by DNA restriction fragment mapping in order to identify clones where the glucoamylase gene was in the same orientation as the promoter; one such clone was selected for further study as pGC24.

In order to examine expression of the glucoamylase gene using the trp promoter, plasmid pGC24 was transformed into E. coli host MH70 which had been obtained from the E. coli Genetic Stock Center, Yale University (their collection number is CGSC 6153). MH70 is a mal⁻ E. coli strain whose genotype is araD139, Δ(argF-lac) 205, flbB5301, ptsF25, relAl?, rpsL150, malQ63, bglR15, deoCl? The malQ mutation is in the amylomaltase gene; a mutation in this gene makes E. coli unable to hydrolyze maltose to glucose.

A sample of the MH70 transformed with pCG24 was deposited with the American Type Culture Collection on December 16, 1983, and has been assigned the ATCC Deposit No. 39,537.

The MH70/pGC24 transformant and strain MH70 were grown at 37°C and 200 rpm in 5 ml of the following medium containing tryptophan at 50 mg/l.

| | |
|---|---|
| 25X Bonner-Vogel Salts | 40 ml |
| Ampicillin | 50 mg |
| Glucose | 2 g |
| Vitamin B1 | 10 mg |
| Casamino Acid | 2 g |
| Water | to 1000 ml |

25X Bonner-Vogel Salts (Methods in Enzymology, XVIIA:5):

| | |
|---|---|
| Glass Distilled Water | 670 ml |
| $MgSO_4 \cdot 7H_2O$ | 5 g |
| Citric Acid$\cdot H_2O$ | 50 g |
| $K_2HPO_4$ | 250 g |
| $NaNH_4HPO_4 \cdot 4H_2O$ | 87.5 g |
| Glass Distilled Water to | 1000 ml |

After overnight incubation, the cells were harvested by centrifugation at 3000g for 5 minutes and resuspended in 5 ml of the same medium but without tryptophan. The cells were then subcultured in 20 mls of the medium without tryptophan to an $A_{660}$ of 0.05-0.07. This culture was grown at 37° C and 250 rpm to an $A_{660}$ of 0.05. The cells were harvested from 10 mls of culture by centrifugation as above and resuspended in 1 ml of sonication buffer (15% sucrose, 50 mM Tris pH 7, 40 mM EDTA). The samples were sonicated for 3 minutes (on pulse) in a cup sonicator (Sonifier Cell Disrupter #350, Branson Sonic Power Co.). The cell lysates were centrifuged for 5 minutes in an Eppendorf Microfuge and the clear supernatants were removed for further analysis. The clear lysates were electrophoresed on an polyacrylamide sodium dodecyl sulfate (SDS) gel and analyzed by Western analysis as described in Example 2C. A protein band of approximately 69,000 molecular weight, the size expected for an unglycosylated form of glucoamylase, was detected in the MH70/pGC24 clear lysate but not in the MH70 lysate.

To further demonstrate that an active glucoamylase enzyme was produced in E. coli, MH70/pGC24 and MH70 were streaked on MacConkey Agar (Difco Co., Detroit, MI 48232) plates containing 1% maltose and incubated overnight at 37° C. The fermentation of maltose results in a pH change in the media that is indicated by a shift from a colorless to red color in the colonies; nonfermenting colonies remain colorless. Since MH70 is malQ⁻, its colonies were colorless. The expression of the A. awamori glucoamylase in MH70/pGC24 permitted the hydrolysis of maltose to glucose and the fermentation of the glucose resulted in red colonies. Therefore, an active glucoamylase is produced in E. coli.

## Claims

**1.** A DNA sequence characterized in that it codes for a glucoamylase protein of a fungus belonging to the filamentous Ascomycetes, the DNA sequence in 5' to 3' direction being the following:

GCG ACC TTG GAT TCA TGG TTG AGC AAC GAA GCG ACC GTG GCT CGT
ACT GCC ATC CTG AAT AAC ATC GGG GCG GAC GGT GCT TGG GTG TCG
GGC GCG GAC TCT GGC ATT GTC GTT GCT AGT CCC AGC ACG GAT AAC
CCG GAC TAC TTC TAC ACC TGG ACT CGC GAC TCT GGT CTC GTC CTC
AAG ACC CTC GTC GAT CTC TTC CGA AAT GGA GAT ACC AGT CTC CTC
TCC ACC ATT GAG AAC TAC ATC TCC GCC CAG GCA ATT GTC CAG GGT
ATC AGT AAC CCC TCT GGT GAT CTG TCC AGC GGC GCT GGT CTC GGT GAA
CCC AAG TTC AAT GTC GAT GAG ACT GCC TAC ACT GGT TCT TGG GGA
CGG CCG CAG CGA GAT GGT CCG GCT CTG AGA GCA ACT GCT ATG ATC
GGC TTC GGG CAA TGG CTG CTT GAC AAT GGC TAC ACC AGC ACC GCA
ACG GAC ATT GTT TGG CCC CTC GTT AGG AAC GAC CTG TCG TAT GTG
GCT CAA TAC TGG AAC CAG ACA GGA TAT GAT CTC TGG GAA GAA GTC
AAT GGC TCG TCT TTC TTT ACG ATT GCT GTG CAA CAC CGC GCC CTT
GTC GAA GGT AGT GCC TTC GCG ACG GCC GTC GGC TCG TCC TGC TCC
TGG TGT GAT TCT CAG GCA CCC GAA ATT CTC TGC TAC CTG CAG TCC
TTC TGG ACC GGC AGC TTC ATT CTG GCC AAC TTC GAT AGC AGC CGT
TCC GGC AAG GAC GCA AAC ACC CTC CTG GGA AGC ATC CAC ACC TTT
GAT CCT GAG GCC GCA TGC GAC GAC TCC ACC TTC CAG CCC TGC TCC
CCG CGC GCG CTC GCC AAC CAC AAG GAG GTT GTA GAC TCT TTC CGC
TCA ATC TAT ACC CTC AAC GAT GGT CTC AGT GAC AGC GAG GCT GTT
GCG GTG GGT CGG TAC CCT GAG GAC ACG TAC TAC AAC GGC AAC CCG
TGG TTC CTG TGC ACC TTG GCT GCC GCA GAG CAG TTG TAC GAT GCT
CTA TAC CAG TGG GAC AAG CAG GGG TCG TTG GAG GTC ACA GAT GTG

TCG CTG GAC TTC TTC AAG GCA CTG TAC AGC GAT GCT GCT ACT GGC
ACC TAC TCT TCG TCC AGT TCG ACT TAT AGT AGC ATT GTA GAT GCC
GTG AAG ACT TTC GCC GAT GGC TTC GTC TCT ATT GTG GAA ACT CAC
GCC GCA AGC AAC GGC TCC ATG TCC GAG CAA TAC GAC AAG TCT GAT
GGC GAG CAG CTT TCC GCT CGC GAC CTG ACC TGG TCT TAT GCT GCT
CTG CTG ACC GCC AAC AAC CGT CGT AAC GTC GTG CCT TCC GCT TCT
TGG GGC GAG ACC TCT GCC AGC AGC GTG CCC GGC ACC TGT GCG GCC
ACA TCT GCC ATT GGT ACC TAC AGC AGT GTG ACT GTC ACC TCG TGG
CCG AGT ATC GTG GCT ACT GGC GGC ACC ACT ACG ACG GCT ACC CCC
ACT GGA TCC GGC AGC GTG ACC TCG ACC AGC AAG ACC ACC GCG ACT
GCT AGC AAG ACC AGC ACC AGT ACG TCA TCA ACC TCC TGT ACC ACT
CCC ACC GCC GTG GCT GTG ACT TTC GAT CTG ACA GCT ACC ACC ACC
TAC GGC GAG AAC ATC TAC CTG GTC GGA TCG ATC TCT CAG CTG GGT
GAC TGG GAA ACC AGC GAC GGC ATA GCT CTG AGT GCT GAC AAG TAC
ACT TCC AGC GAC CCG CTC TGG TAT GTC ACT GTG ACT CTG CCG GCT
GGT GAG TCG TTT GAG TAC AAG TTT ATC CGC ATT GAG AGC GAT GAC
TCC GTG GAG TGG GAG AGT GAT CCC AAC CGA GAA TAC ACC GTT CCT
CAG GCG TGC GGA ACG TCG ACC GCG ACG GTG ACT GAC ACC TGG CGG

or mutants thereof coding for a glucoamylase protein with the same functional properties wherein said DNA sequence is free of introns and is capable, when correctly combined with a cleaved expression vector, of expressing a protein having glucoamylase enzyme activity upon transformation of a yeast host organism by the vector, said protein being not produced naturally or natively by the yeast host organism.

2. A DNA sequence of claim 1 wherein the DNA is cDNA and wherein the fungal glucoamylase protein for which the sequence codes is from the genus Aspergillus (species A. awamori or A. niger).

3. A DNA sequence of claim 2 wherein the fungal glucoamylase protein for which the DNA sequence codes is from the genus Aspergillus (species A. awamori or A. niger).

4. A DNA sequence of claim 3 wherein the fungal glucoamylase protein for which the DNA sequence codes is from A. awamori NRRL 15,271.

5. A DNA expression vector characterized in that it contains a promoter fragment, that functions in a yeast host and a DNA segment having a DNA sequence according to anyone of claims 1 to 4, said DNA segment being in an orientation with the promoter fragment such that in the yeast host it is expressed to produce a non-native glucoamylase protein.

6. The vector of claim 5 wherein the promoter fragment is derived from the yeast ENO1 gene and wherein the vector further comprises an ENO-1 terminator fragment.

7. The vector of claim 5 or 6 further comprising Leu-2 as a selectable marker.

8. The vector of any one of claims 5 to 7 wherein the yeast is a species of the genus Saccharomyces.

9. A yeast host organism transformed by an expression vector according to anyone of claims 5 to 8.

10. The yeast host according to claim 9 which is the S. cerevisiae strain C468.

11. A process for producing glucoamylase characterized by growing the host organism of claim 9 or 10 in a culture medium.

12. A process for the saccharification of starch or starch hydrolysates containing oligosaccharides characterized by treating said starch or starch hydrolysate with a yeast host transformed by a DNA expression vector of any one of claims 5 to 8 or with a glucoamylase enzyme preparation wherein said preparation has been produced by a yeast host organism transformed by a DNA expression vector of any one of claims 5 to 8.

13. A process for producing ethanol by simultaneous saccharification and fermentation characterized by growing, on a nonfermentable carbon source which is a substrate for glucoamylase, a yeast host organism transformed by a DNA expression vector of anyone of claims 5 to 8.

14. The process of claim 13 wherein the yeast is a species of the genus Saccharomyces.

## Revendications

1. Séquence d'ADN, caractérisée en ce qu'elle code pour une protéine de type glucoamylase d'un champignon appartenant aux ascomycètes filamenteux, la séquence d'ADN étant la suivante selon la direction 5'-3' :

GCG ACC TTG GAT TCA TGG TTG AGC AAC GAA GCG ACC GTG GCT CGT
ACT GCC ATC CTG AAT AAC ATC GGG GCG GAC GGT GCT TGG GTG TCG
GGC GCG GAC TCT GGC ATT GTC GTT GCT AGT CCC AGC ACG GAT AAC
CCG GAC TAC TTC TAC ACC TGG ACT CGC GAC TCT GGT CTC GTC CTC
AAG ACC CTC GTC GAT CTC TTC CGA AAT GGA GAT ACC AGT CTC CTC
TCC ACC ATT GAG AAC TAC ATC TCC GCC CAG GCA ATT GTC CAG GGT
ATC AGT AAC CCC TCT GGT GAT CTG TCC AGC GGC GCT GGT CTC GGT GAA
CCC AAG TTC AAT GTC GAT GAG ACT GCC TAC ACT GGT TCT TGG GGA
CGG CCG CAG CGA GAT GGT CCG GCT CTG AGA GCA ACT GCT ATG ATC
GGC TTC GGG CAA TGG CTG CTT GAC AAT GGC TAC ACC AGC ACC GCA
ACG GAC ATT GTT TGG CCC CTC GTT AGG AAC GAC CTG TCG TAT GTG
GCT CAA TAC TGG AAC CAG ACA GGA TAT GAT CTC TGG GAA GAA GTC
AAT GGC TCG TCT TTC TTT ACG ATT GCT GTG CAA CAC CGC GCC CTT
GTC GAA GGT AGT GCC TTC GCG ACG GCC GTC GGC TCG TCC TGC TCC
TGG TGT GAT TCT CAG GCA CCC GAA ATT CTC TGC TAC CTG CAG TCC
TTC TGG ACC GGC AGC TTC ATT CTG GCC AAC TTC GAT AGC AGC CGT
TCC GGC AAG GAC GCA AAC ACC CTC CTG GGA AGC ATC CAC ACC TTT
GAT CCT GAG GCC GCA TGC GAC GAC TCC ACC TTC CAG CCC TGC TCC
CCG CGC GCG CTC GCC AAC CAC AAG GAG GTT GTA GAC TCT TTC CGC
TCA ATC TAT ACC CTC AAC GAT GGT CTC AGT GAC AGC GAG GCT GTT
GCG GTG GGT CGG TAC CCT GAG GAC ACG TAC TAC AAC GGC AAC CCG
TGG TTC CTG TGC ACC TTG GCT GCC GCA GAG CAG TTG TAC GAT GCT
CTA TAC CAG TGG GAC AAG CAG GGG TCG TTG GAG GTC ACA GAT GTG
TCG CTG GAC TTC TTC AAG GCA CTG TAC AGC GAT GCT GCT ACT GGC
ACC TAC TCT TCG TCC AGT TCG ACT TAT AGT AGC ATT GTA GAT GCC
GTG AAG ACT TTC GCC GAT GGC TTC GTC TCT ATT GTG GAA ACT CAC
GCC GCA AGC AAC GGC TCC ATG TCC GAG CAA TAC GAC AAG TCT GAT
GGC GAG CAG CTT TCC GCT CGC GAC CTG ACC TGG TCT TAT GCT GCT

CTG CTG ACC GCC AAC AAC CGT CGT AAC GTC GTG CCT TCC GCT TCT
TGG GGC GAG ACC TCT GCC AGC AGC GTG CCC GGC ACC TGT GCG GCC
ACA TCT GCC ATT GGT ACC TAC AGC AGT GTG ACT GTC ACC TCG TGG
CCG AGT ATC GTG GCT ACT GGC GGC ACC ACT ACG ACG GCT ACC CCC
ACT GGA TCC GGC AGC GTG ACC TCG ACC AGC AAG ACC ACC GCG ACT
GCT AGC AAG ACC AGC ACC AGT ACG TCA TCA ACC TCC TGT ACC ACT
CCC ACC GCC GTG GCT GTG ACT TTC GAT CTG ACA GCT ACC ACC ACC
TAC GGC GAG AAC ATC TAC CTG GTC GGA TCG ATC TCT CAG CTG GGT
GAC TGG GAA ACC AGC GAC GGC ATA GCT CTG AGT GCT GAC AAG TAC
ACT TCC AGC GAC CCG CTC TGG TAT GTC ACT GTG ACT CTG CCG GCT
GGT GAG TCG TTT GAG TAC AAG TTT ATC CGC ATT GAG AGC GAT GAC
TCC GTG GAG TGG GAG AGT GAT CCC AAC CGA GAA TAC ACC GTT CCT
CAG GCG TGC GGA ACG TCG ACC GCG ACG GTG ACT GAC ACC TGG CGG

ou des mutants de celle-ci, codant pour une protéine de type glucoamylase ayant les mêmes propriétés fonctionnelles, cette séquence d'ADN étant exempte d'introns, et capable, lorsqu'elle est correctement associée à un vecteur d'expression clivé, d'exprimer une protéine ayant une activité enzymatique de glucoamylase, par transformation d'une levure formant organisme-hôte par le vecteur, cette protéine n'étant pas native ou produite naturellement dans la levure formant organisme-hôte.

2. Séquence d'ADN selon la revendication 1, dans laquelle l'ADN est un ADNc, et dans laquelle la protéine fongique de type glucoamylase, pour laquelle la séquence code, est dérivée du genre Aspergillus (l'espèce A. awamori ou A. niger).

3. Séquence d'ADN selon la revendication 2, dans laquelle la protéine fongique de type glucoamylase pour laquelle la séquence d'ADN code, est dérivée du genre Aspergillus (l'espèce A. awamori ou A. niger).

4. Séquence d'ADN selon la revendication 3, dans laquelle la protéine fongique de type glucoamylase pour laquelle code la séquence d'ADN, est dérivée de A. awamori NRRL 15 271.

5. Vecteur d'expression d'ADN, caractérisé en ce qu'il contient un fragment promoteur qui fonctionne dans une levure-hôte, et un segment d'ADN comportant une séquence d'ADN selon l'une quelconque des revendications 1 à 4, ce segment d'ADN étant orienté par rapport au fragment promoteur, de telle façon qu'il s'exprime dans la levure-hôte, en produisant une protéine non native de type glucoamylase.

6. Vecteur selon la revendication 5, dans lequel le fragment promoteur est dérivé du gène ENO1 de levure, et comprend en outre un fragment terminateur ENO-1.

7. Vecteur selon la revendication 5 ou 6, comprenant en outre le marqueur sélectionnable Leu-2.

8. Vecteur selon l'une quelconque des revendications 5 à 7, dans lequel la levure est une espèce du genre Saccharomyces.

9. Levure formant organisme-hôte, transformée par un vecteur d'expression selon l'une quelconque des revendications 5 à 8.

10. Levure-hôte selon la revendication 9, qui est la souche C468 de S. cerevisiae.

11. Procédé de production de glucoamylase, caractérisé en ce qu'on cultive l'organisme-hôte selon la revendication 9 ou 10, dans un milieu de culture.

12. Procédé de saccharification d'amidon ou d'hydrolysats d'amidon contenant des oligosaccharides, caractérisé en ce qu'on traite l'amidon ou l'hydrolysat d'amidon, avec une levure-hôte transformée par un vecteur d'expression d'ADN selon l'une quelconque des revendications 5 à 8, ou avec une préparation à base d'enzyme glucoamylase produite par une levure formant organisme-hôte, transformée par un vecteur d'expression d'ADN selon l'une quelconque des revendications 5 à 8.

13. Procédé de production d'éthanol par saccharification et fermentation simultanées, caractérisé en ce qu'on cultive, sur une source de carbone non fermentable constituant un substrat de la glucoamylase, une levure formant organisme-hôte transformée par un vecteur d'expression d'ADN selon l'une quelconque des revendications 5 à 8.

14. Procédé selon la revendication 13, dans lequel la levure est une espèce du genre Saccharomyces.

## Ansprüche

1. DNA-Sequenz, **dadurch gekennzeichnet**, daß sie für ein Glucoamylaseprotein eines zu den filamentösen Ascomyceten gehörenden Pilzes codiert, wobei folgende DNA-Sequenz in 5'- nach 3'-Richtung vorhanden ist:

GCG ACC TTG GAT TCA TGG TTG AGC AAC GAA GCG ACC GTG GCT CGT
ACT GCC ATC CTG AAT AAC ATC GGG GCG GAC GGT GCT TGG GTG TCG
GGC GCG GAC TCT GGC ATT GTC GTT GCT AGT CCC AGC ACG GAT AAC
CCG GAC TAC TTC TAC ACC TGG ACT CGC GAC TCT GGT CTC GTC CTC
AAG ACC CTC GTC GAT CTC TTC CGA AAT GGA GAT ACC AGT CTC CTC
TCC ACC ATT GAG AAC TAC ATC TCC GCC CAG GCA ATT GTC CAG GGT
ATC AGT AAC CCC TCT GGT GAT CTG TCC AGC GGC GCT GGT CTC GGT GAA
CCC AAG TTC AAT GTC GAT GAG ACT GCC TAC ACT GGT TCT TGG GGA
CGG CCG CAG CGA GAT GGT CCG GCT CTG AGA GCA ACT GCT ATG ATC
GGC TTC GGG CAA TGG CTG CTT GAC AAT GGC TAC ACC AGC ACC GCA
ACG GAC ATT GTT TGG CCC CTC GTT AGG AAC GAC CTG TCG TAT GTG
GCT CAA TAC TGG AAC CAG ACA GGA TAT GAT CTC TGG GAA GAA GTC
AAT GGC TCG TCT TTC TTT ACG ATT GCT GTG CAA CAC CGC GCC CTT
GTC GAA GGT AGT GCC TTC GCG ACG GCC GTC GGC TCG TCC TGC TCC
TGG TGT GAT TCT CAG GCA CCC GAA ATT CTC TGC TAC CTG CAG TCC
TTC TGG ACC GGC AGC TTC ATT CTG GCC AAC TTC GAT AGC AGC CGT
TCC GGC AAG GAC GCA AAC ACC CTC CTG GGA AGC ATC CAC ACC TTT
GAT CCT GAG GCC GCA TGC GAC GAC TCC ACC TTC CAG CCC TGC TCC

CCG CGC GCG CTC GCC AAC CAC AAG GAG GTT GTA GAC TCT TTC CGC

TCA ATC TAT ACC CTC AAC GAT GGT CTC AGT GAC AGC GAG GCT GTT

GCG GTG GGT CGG TAC CCT GAG GAC ACG TAC TAC AAC GGC AAC CCG

TGG TTC CTG TGC ACC TTG GCT GCC GCA GAG CAG TTG TAC GAT GCT

CTA TAC CAG TGG GAC AAG CAG GGG TCG TTG GAG GTC ACA GAT GTG

TCG CTG GAC TTC TTC AAG GCA CTG TAC AGC GAT GCT GCT ACT GGC

ACC TAC TCT TCG TCC AGT TCG ACT TAT AGT AGC ATT GTA GAT GCC

GTG AAG ACT TTC GCC GAT GGC TTC GTC TCT ATT GTG GAA ACT CAC

GCC GCA AGC AAC GGC TCC ATG TCC GAG CAA TAC GAC AAG TCT GAT

GGC GAG CAG CTT TCC GCT CGC GAC CTG ACC TGG TCT TAT GCT GCT

CTG CTG ACC GCC AAC AAC CGT CGT AAC GTC GTG CCT TCC GCT TCT

TGG GGC GAG ACC TCT GCC AGC AGC GTG CCC GGC ACC TGT GCG GCC

ACA TCT GCC ATT GGT ACC TAC AGC AGT GTG ACT GTC ACC TCG TGG

CCG AGT ATC GTG GCT ACT GGC GGC ACC ACT ACG ACG GCT ACC CCC

ACT GGA TCC GGC AGC GTG ACC TCG ACC AGC AAG ACC ACC GCG ACT

GCT AGC AAG ACC AGC ACC AGT ACG TCA TCA ACC TCC TGT ACC ACT

CCC ACC GCC GTG GCT GTG ACT TTC GAT CTG ACA GCT ACC ACC ACC

TAC GGC GAG AAC ATC TAC CTG GTC GGA TCG ATC TCT CAG CTG GGT

GAC TGG GAA ACC AGC GAC GGC ATA GCT CTG AGT GCT GAC AAG TAC

ACT TCC AGC GAC CCG CTC TGG TAT GTC ACT GTG ACT CTG CCG GCT

GGT GAG TCG TTT GAG TAC AAG TTT ATC CGC ATT GAG AGC GAT GAC

TCC GTG GAG TGG GAG AGT GAT CCC AAC CGA GAA TAC ACC GTT CCT

CAG GCG TGC GGA ACG TCG ACC GCG ACG GTG ACT GAC ACC TGG CGG

oder Mutanten davon, die für ein Glucoamylaseprotein mit den gleichen funktionalen Eigenschaften codieren, wobei die DNA-Sequenz keine Introns aufweist und befähigt ist, wenn sie mit einem gespaltenen Expressionsvektor richtig kombiniert wird, nach der Transformation eines Hefe-Wirtsorganismus durch den Vektor ein Protein mit Glucoamylase-Enzymaktivität zu exprimieren, welches Protein nicht natürlich oder nativ durch den Hefe-Wirtsorganismus erzeugt wird.

2. DNA-Sequenz nach Anspruch 1, wobei die DNA eine cDNA ist und das Glucoamylaseprotein des Pilzes, für das die Sequenz codiert, aus der Gattung Aspergillus (Arten A. awamori oder A. niger) stammt.

3. DNA-Sequenz nach Anspruch 2, wobei das Glucoamylaseprotein des Pilzes, für das die DNA-Sequenz codiert, aus der Gattung Aspergillus (Arten A. awamori oder A. niger) stammt.

4. DNA-Sequenz nach Anspruch 3, wobei das Glucoamylaseprotein des Pilzes, für das die DNA-Sequenz codiert, aus A. awamori NRRL 15,271 stammt.

5. DNA-Expressionsvektor, **dadurch gekennzeichnet**, daß er ein Promotorfragment, das in einem Hefewirt funktioniert, und einen DNA-Abschnitt, der eine DNA-Sequenz nach einem der Ansprüche 1 bis 4 aufweist, enthält, wobei der DNA-Abschnitt mit dem Promotorfragment so orientiert ist, daß er in einem Hefewirt unter Erzeugung eines nicht-nativen Glucoamylaseproteins exprimiert wird.

6. Vektor nach Anspruch 5, wobei das Promotorfragment aus dem Hefe ENO1-Gen stammt und der Vektor weiter ein ENO1-Terminationsfragment umfaßt.

7. Vektor nach Anspruch 5 oder 6, der weiter Leu-2 als einen selektierbaren Marker umfaßt.

8. Vektor nach einem der Ansprüche 5 bis 7, wobei die Hefe eine Art aus der Gattung Saccharomyces ist.

9. Hefe-Wirtsorganismus, der durch einen Expressionsvektor nach einem der Ansprüche 5 bis 8 transformiert ist.

10. Hefewirt nach Anspruch 9, der S. cerevisiae Stamm C468 ist.

11. Verfahren zur Herstellung von Glucoamylase, **gekennzeichnet durch** das Züchten des Wirtsorganismus nach Anspruch 9 oder 10 in einem Kulturmedium.

12. Verfahren zur Zuckerbildung aus Stärke oder Oligosaccharide enthaltenden Stärkehydrolysaten, **gekennzeichnet durch** die Behandlung der Stärke oder des Stärkehydrolysats mit einem Hefewirt, der durch einen DNA-Expressionsvektor nach einem der Ansprüche 5 bis 8 transformiert ist, oder mit einer Glucoamylase-Enzympräparation, wobei die Präparation von einem Hefe-Wirtsorganismus, der durch einen DNA-Expressionsvektor nach einem der Ansprüche 5 bis 8 transformiert ist, hergestellt worden ist.

13. Verfahren zur Herstellung von Ethanol durch gleichzeitige Zuckerbildung und Fermentation, **gekennzeichnet durch** das Züchten eines Hefe-Wirtsorganismus, der durch einen DNA-Expressionsvektor nach einem der Ansprüche 5 bis 8 transformiert ist, in einer nicht-fermentierbaren Kohlenstoffquelle, die ein Substrat für die Glucoamylase ist.

14. Verfahren nach Anspruch 13, wobei die Hefe eine Art der Gattung Saccharomyces ist.

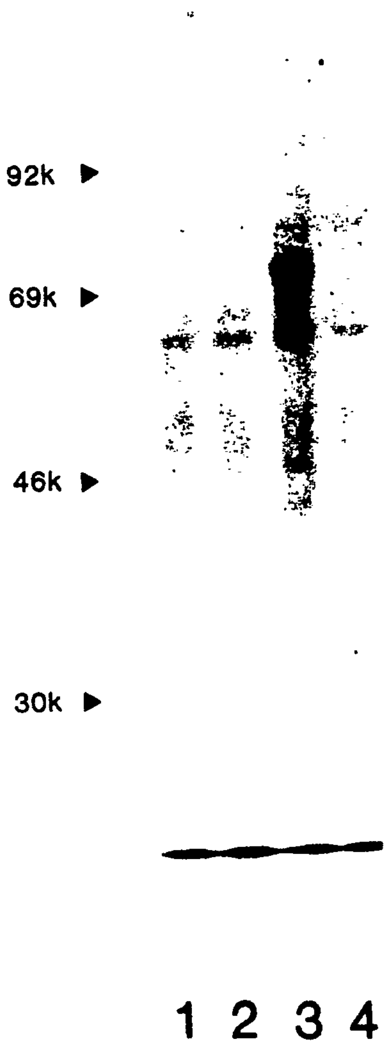


FIG. I

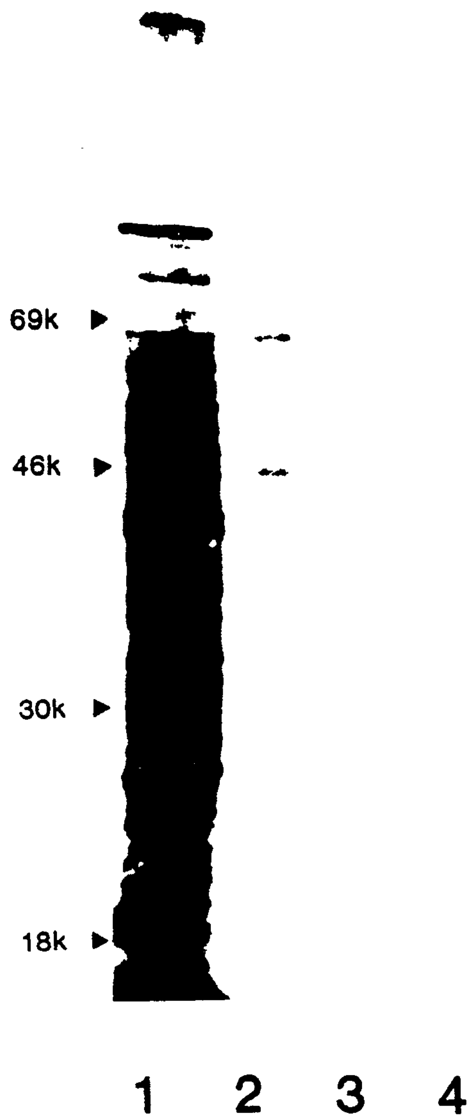


FIG. 4

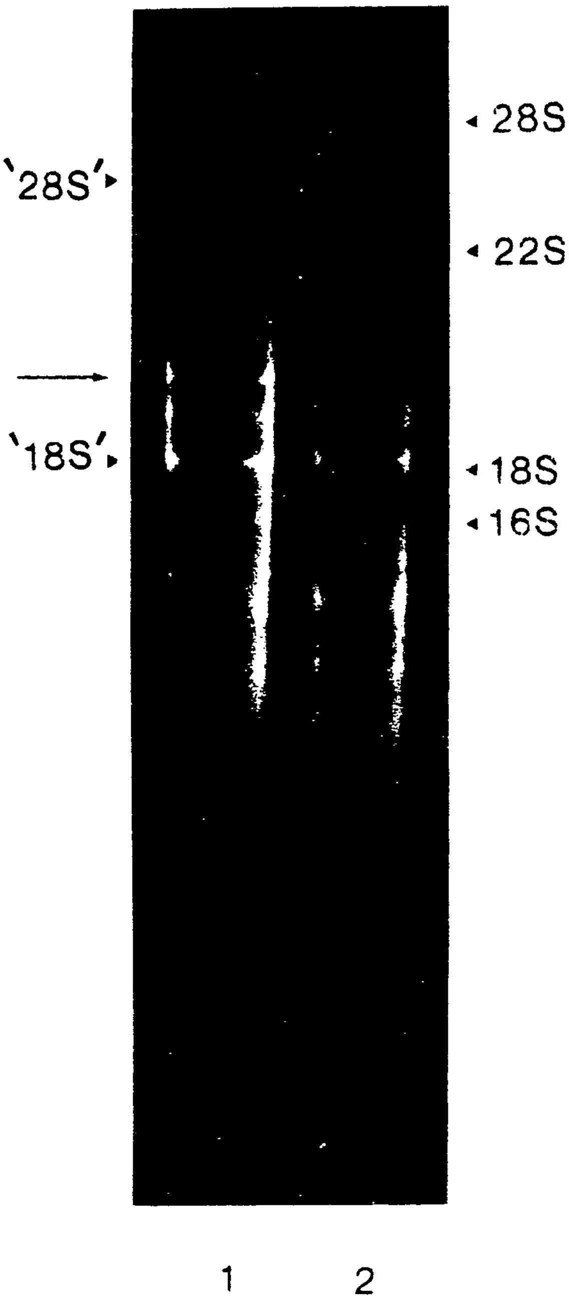


FIG. 2A

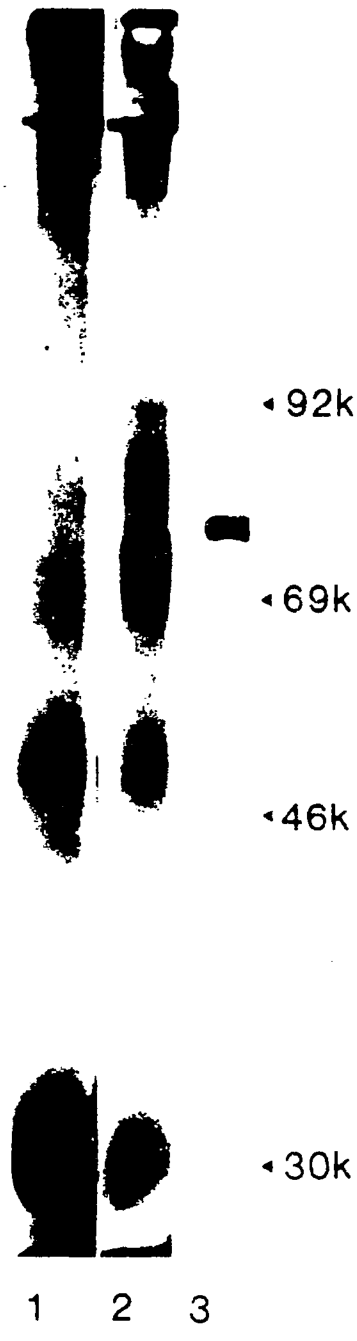


FIG. 2B

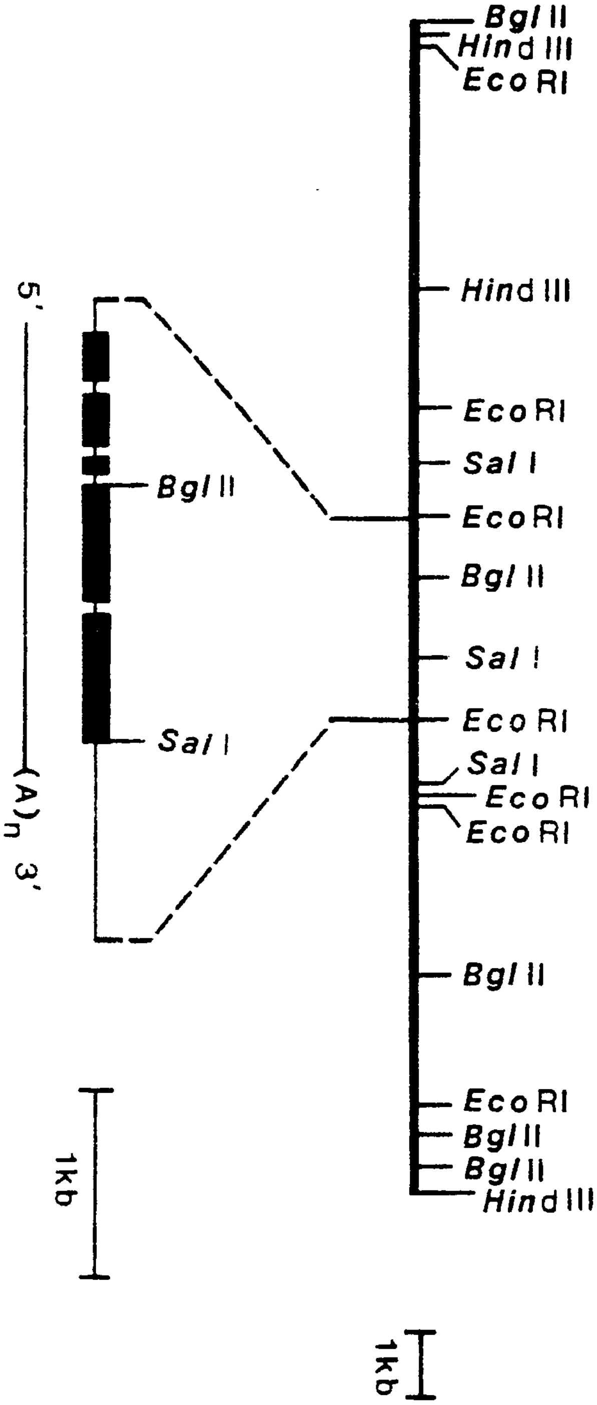
Bgl II
Hind III
Eco RI
Hind III
Eco RI
Sal I
Eco RI
Bgl II
Sal I
Eco RI
Sal I
Eco RI
Eco RI
Bgl II
Eco RI
Bgl II
Bgl II
Hind III
1kb
5'
Bgl II
Sal I
(A)n 3'
1kb

FIG. 3

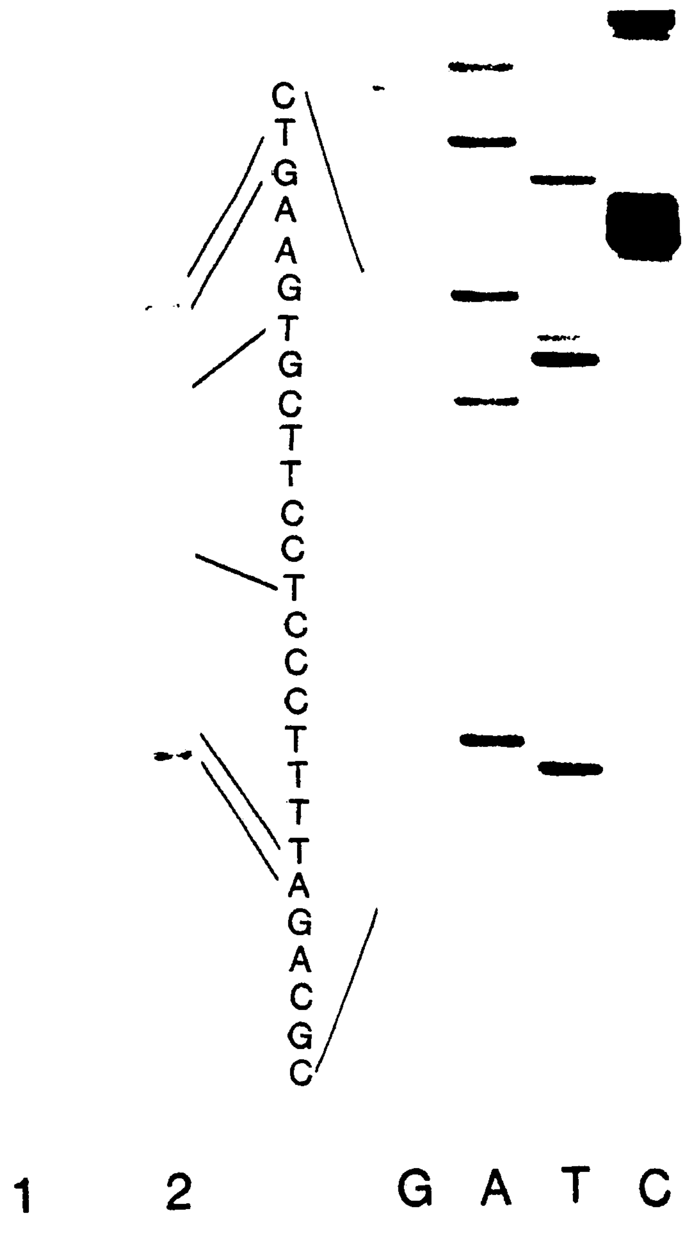


FIG. 5

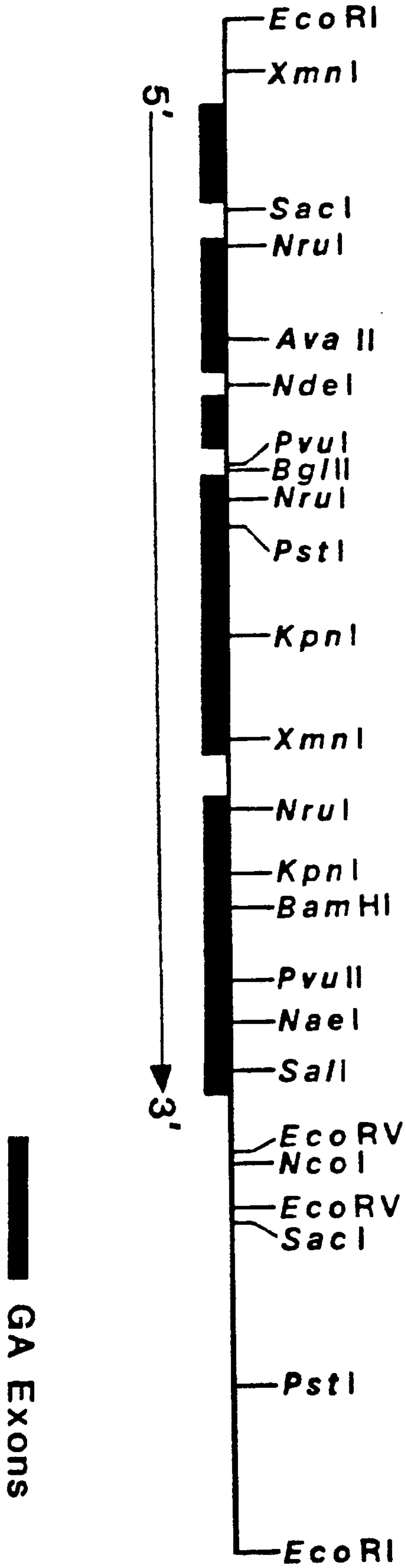


FIG. 6
Genomic Clone

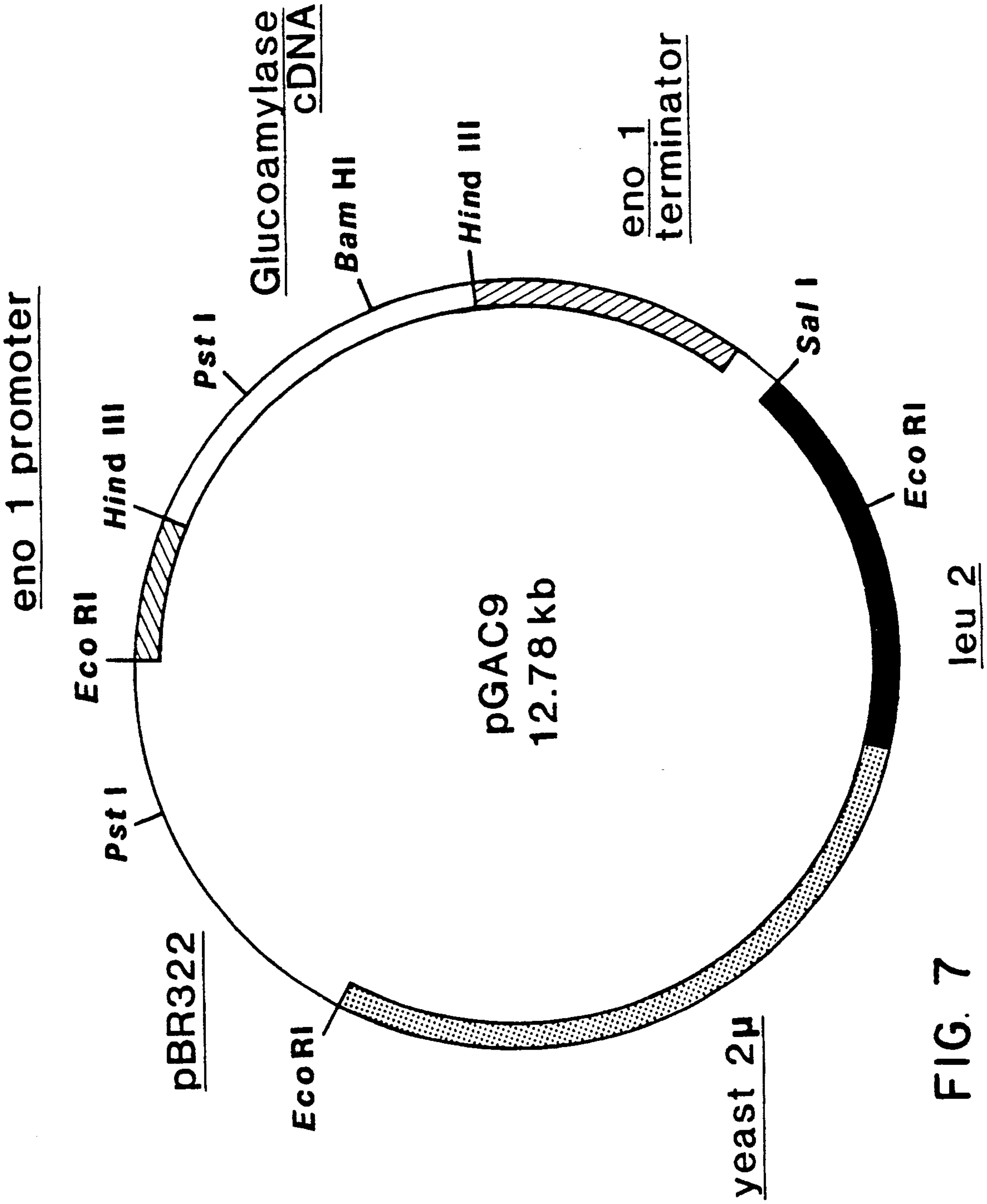
eno 1 promoter
Glucoamylase cDNA
Bam HI
Hind III
eno 1 terminator
Pst I
Hind III
Sal I
Eco RI
Eco RI
pGAC9
12.78 kb
leu 2
Pst I
pBR322
Eco RI
yeast 2μ

FIG. 7

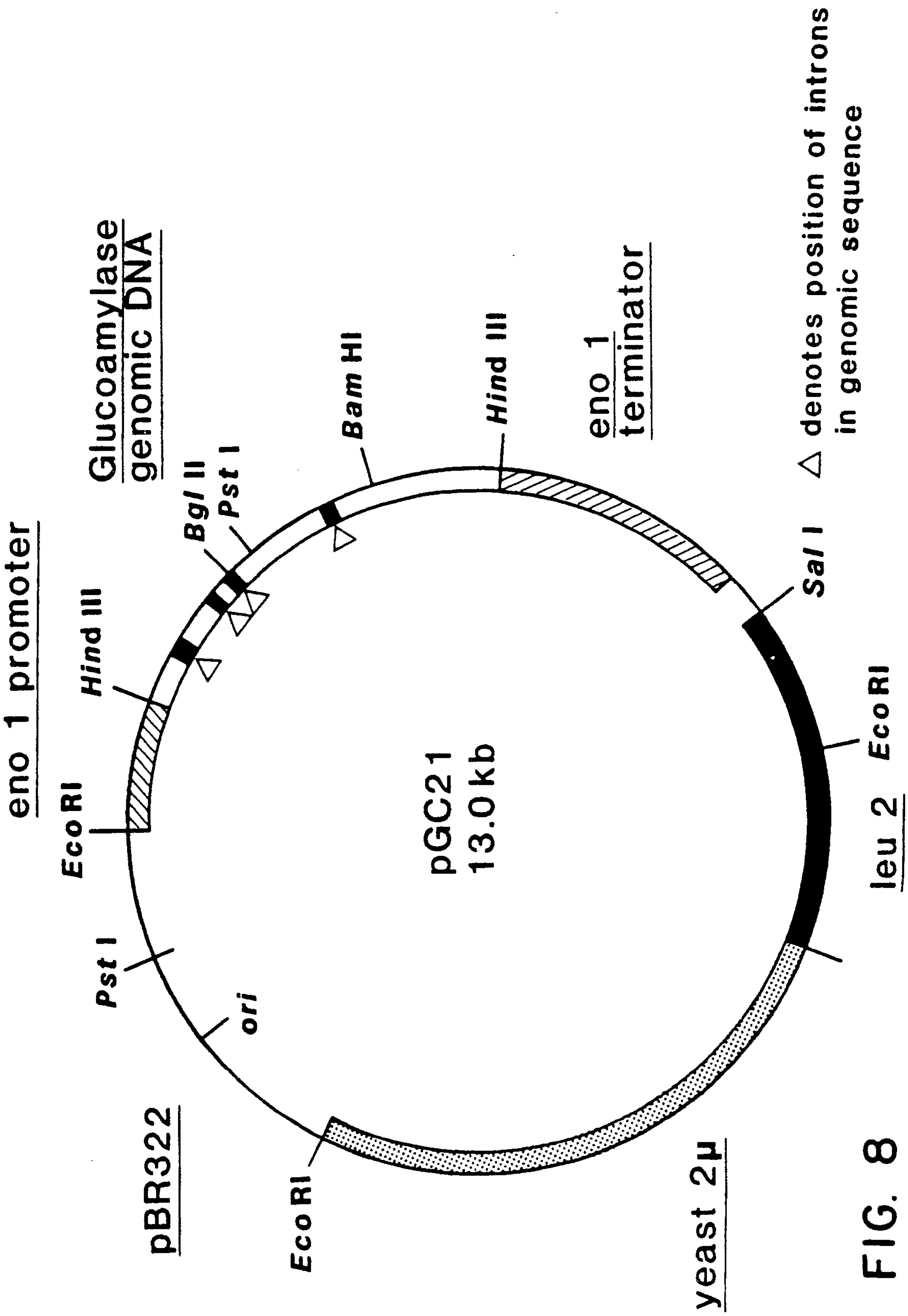

eno 1 promoter
Glucoamylase genomic DNA
pBR322
Pst I
EcoRI
Hind III
Bgl II
Pst I
Bam HI
Hind III
eno 1 terminator
Sal I
EcoRI
leu 2
ori
EcoRI
yeast 2μ
pGC21
13.0 kb
△ denotes position of introns in genomic sequence


FIG. 8

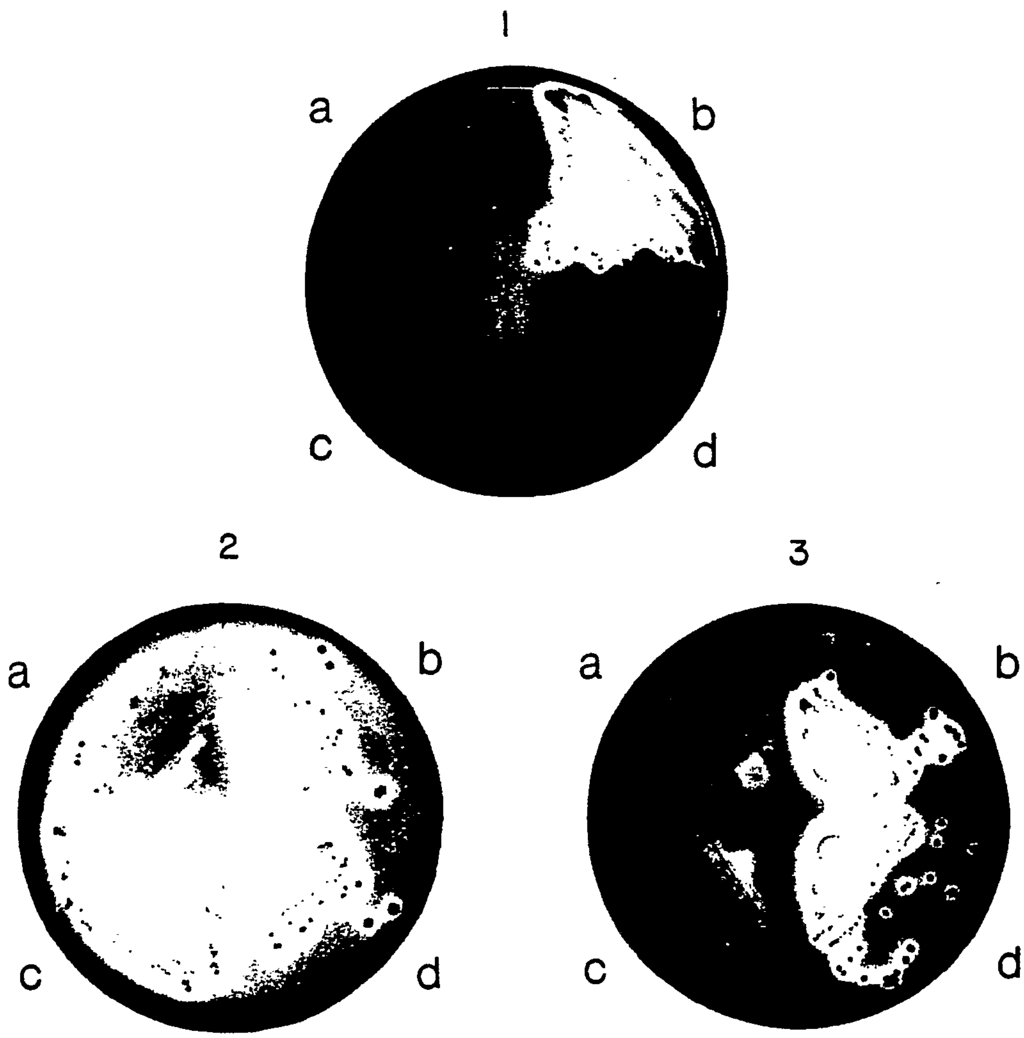


FIG. 9

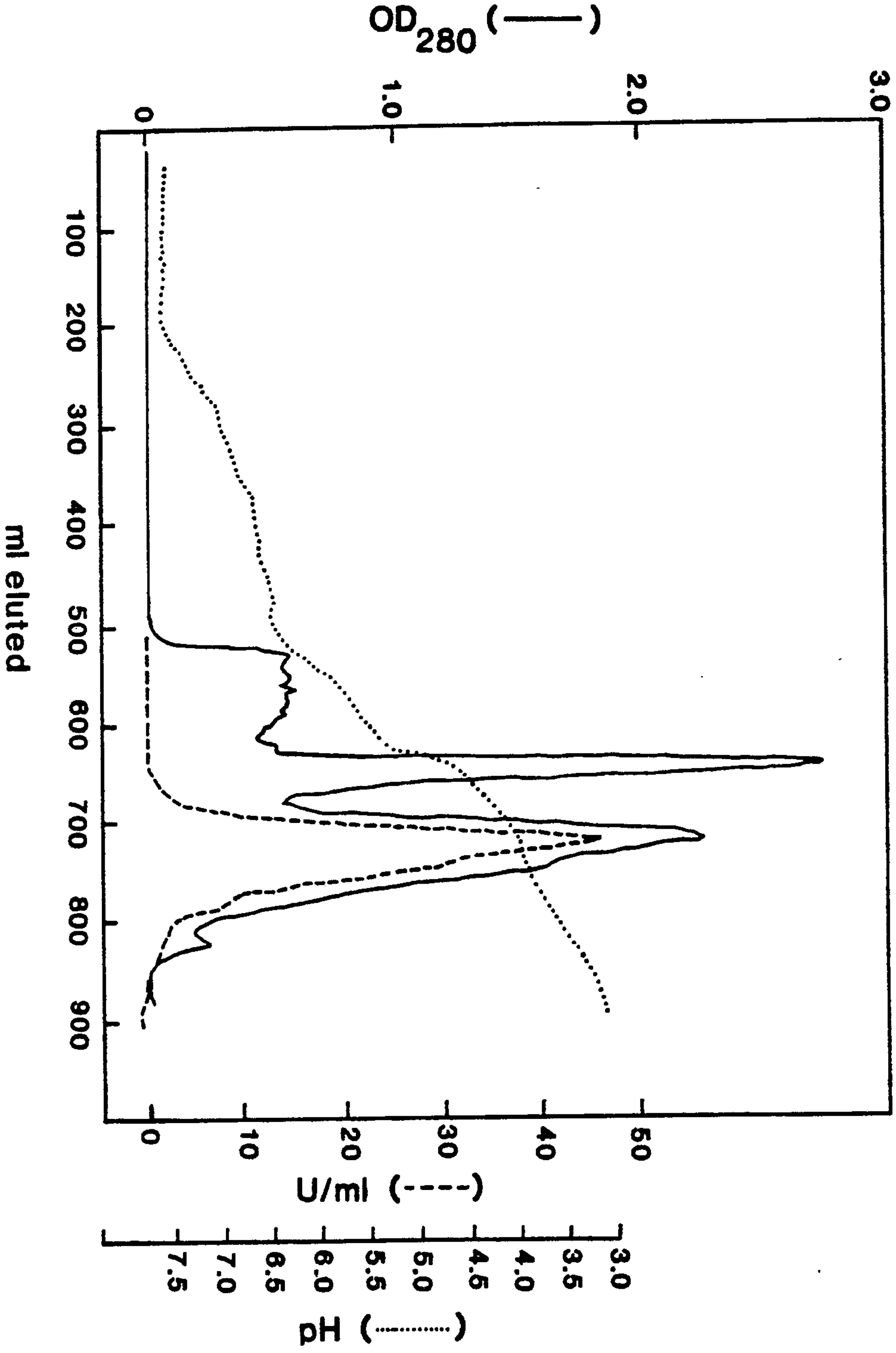
OD280 (——)
0
1.0
2.0
3.0
100
200
300
400
500
600
700
800
900
ml eluted
0
10
20
30
40
50
U/ml (----)
7.5
7.0
6.5
6.0
5.5
5.0
4.5
4.0
3.5
3.0
pH (..........)

FIG. 10

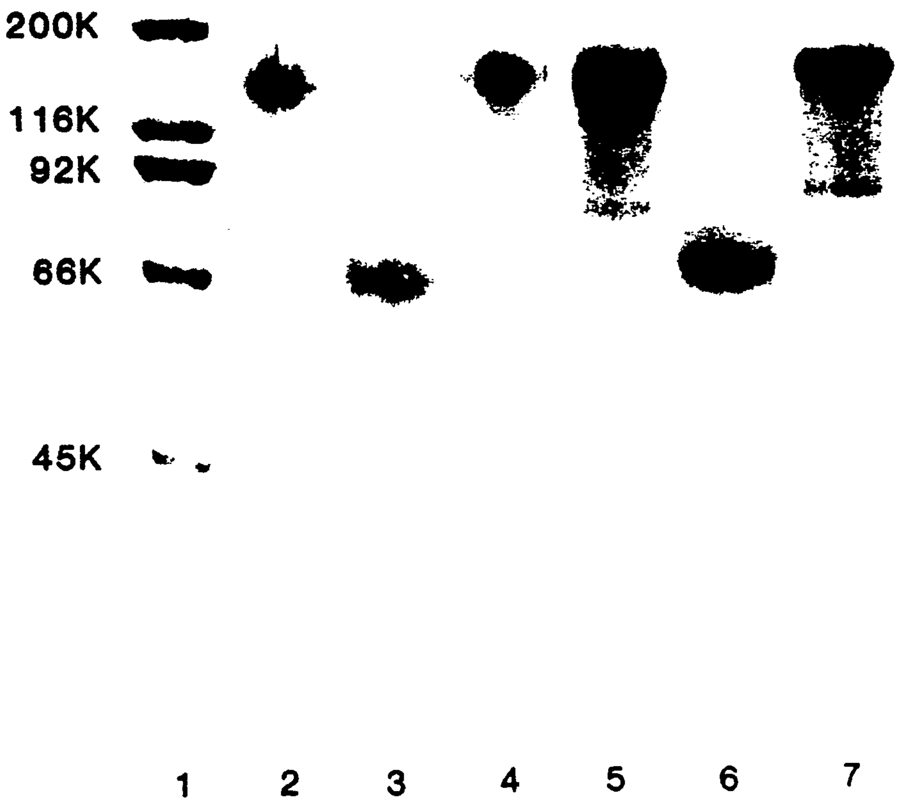


FIG. 11